# EUROPEAN PATENT APPLICATION

(11) **EP 4 613 195 A1**
(43) Date of publication of application: **10.09.2025**
(21) Application number: 25161283.4
(22) Date of filing: 03.03.2025
(51) Int. Cl.: A61B 5/15, A61B 5/151

(54) **DERMAL PATCH WITH BIOMETRIC SENSOR**

(30) Priority: 05.03.2024 US 202418596098
(71) Applicant: Satio, Inc., Boston, Massachusetts 02210 (US)
(72) Inventor: Nawana, Namal, Weston, MA 02493 (US); Al-Shamsie, Ziad Tarik, San Diego, CA 92103 (US); Moniz, Mike, Boston, MA 02210 (US)
(74) Representative: Grünecker Patent- und Rechtsanwälte PartG mbB

(57) **Abstract**

A dermal patch system for collecting a physiological sample from a subject includes a cartridge configured to attach to the skin of the subject, and a lancet configured to engage with the cartridge and draw a physiological sample upon engagement. The cartridge includes a biometric sensor configured to obtain biometric data from a subject.

## Description

### RELATED APPLICATIONS

The present application claims priority to U.S. Application No. 17/719,881 filed on April 13, 2022, which claims priority to U.S. Provisional Patent Application No. 63/174,956 filed on April 14, 2021, U.S. Application No. 17/412,205 filed on August 25, 2021, which claims priority to U.S. Provisional Patent Application No. 63/190,700 filed on May 19, 2021 and U.S. Provisional Patent Application No. 63/174,956 filed on April 14, 2021, U.S. Application No. 17/747,544 filed on May 18, 2022, which claims priority to U.S. Provisional Patent Application No. 63/190,700 filed on May 19, 2021, U.S. Application No. 17/903,802 filed on September 6, 2022, U.S. Application No. 18/090,026 filed on December 28, 2022, U.S. Application No. 17/971,142 filed on October 21, 2022, U.S. Application No. 17/991,284 filed on November 21, 2022 and U.S. Application No. 18/090,063 filed on December 28, 2022, which are hereby incorporated by reference herein in their entireties.

### TECHNICAL FIELD

The present teachings are generally directed to a lancet for use with a cartridge of a dermal patch system (also referred to as "a dermal patch" herein) and a dermal patch system that includes a biometric sensor for identification of a subject from whom a physiological sample is drawn for analysis.

### BACKGROUND

Several diagnostic tests may be performed by analyzing a physiological sample from a subject. In order to obtain the physiological sample, a subject may have to travel to a diagnostic laboratory, where a medical professional obtains the sample with a standard syringe. This process may be time consuming and burdensome to the subject.

### SUMMARY

Aspects of the present disclosure address the above-referenced problems and/or others.

In one aspect, a dermal patch system for collecting a physiological sample from a subject includes a cartridge configured to attach to the skin of the subject and a lancet configured to engage with the cartridge and draw a physiological sample upon engagement. The cartridge includes a biometric sensor, such as a fingerprint scanner, configured to obtain biometric data, e.g., fingerprint data, from a subject. In some embodiments, the cartridge further includes a computer system configured to receive the biometric data, e.g., the fingerprint data, from the biometric scanner and store the biometric data within a memory of the computer system. In some embodiments, the cartridge further includes a power supply configured to power the biometric sensor and the computer system. In some embodiments, the power supply is configured to be activated to supply electrical power to the biometric sensor and the computer system when the lancet engages with the cartridge. By way of example, in some embodiments, in absence of the engagement of the lancet with the cartridge, the electrical connection between the power supply and the biometric sensor is in the form of an open circuit such that the engagement of the lancet with the cartridge closes the electrical circuit to allow the power supply to provide electrical power to the biosensor and the computer system In some such embodiments, the cartridge includes first electrical contacts and the lancet includes second electrical contacts, wherein the engagement of the lancet with the cartridge generates an electrical path between the first and second electrical contacts to close the circuit, e.g., the engagement of the lancet with the cartridge can result in physical contact between . By way of example, in some embodiments, the first and second electrical contacts are pogo style connectors. In some embodiments, the first electrical contacts are electrically conductive pads and the second electrical contacts are pogo style connectors. In some such embodiments, the pogo style connectors of the lancet can engage with the conductive pads of the cartridge when the lancet is received by the cartridge.

In some embodiments, the lancet includes a needle and the cartridge includes a lancet receiving element that is configured to automatically cause the lancet to deploy the needle to draw a physiological sample upon engagement of the cartridge with the lancet. In some embodiments, the lancet is configured to automatically retract the needles. In some embodiments, the lancet receiving element includes the first electrical contacts and a portion of the lancet that engages with the lancet receiving element includes the second electrical contacts. In some embodiments, the power supply is a battery or a charged capacitor. In some embodiments, the power supply is a charged capacitor that automatically powers the biometric sensor and the computer system when the lancet engages with the cartridge. In some embodiments, the cartridge is configured to receive and store the physiological sample.

In another aspect, a method for obtaining a physiological sample from a subject includes affixing a cartridge of a dermal patch system to the subject's skin, engaging a lancet with the cartridge to draw a physiological sample from the subject, and using a biometric sensor, e.g., a fingerprint scanner, of the cartridge to obtain biometric data of the subject from whom the physiological sample is drawn. In some embodiments, the method further includes storing the drawn physiological sample within the cartridge. In some embodiments, the method also includes storing the biometric data in a memory of a computer system that is disposed within the cartridge. In some embodiments, the cartridge includes a power supply that is configured to power the biometric sensor and the computer system upon engagement of the lancet with the cartridge. In some embodiments, the power supply is a charged capacitor and engaging the lancet with the cartridge closes a circuit which causes the charged capacitor to automatically power the biometric sensor and the computer system. In some embodiments, the method also includes determining if chain of custody can be established by comparing the fingerprint data stored in the memory of the computer system to other fingerprint data. In certain applications, such as drug testing, it is desirable to validate the chain of custody of a sample under analysis

### BRIEF DESCRIPTION OF THE DRAWINGS

Aspects of the present disclosure may take form in various components and arrangements of components, and in various steps and arrangements of steps. The drawings are only for illustration purpose of preferred embodiments of the present disclosure and are not to be considered as limiting.

Features of embodiments of the present disclosure will be more readily understood from the following detailed description take in conjunction with the accompanying drawings in which:
**Fig. 1** depicts a dermal patch system in accordance with an exemplary embodiment of the present disclosure;
**Fig. 2** depicts a lancet with three needles in accordance with an exemplary embodiment of the present disclosure;
**Fig. 3** is a cross sectional view of the lancet, wherein the lancet is in an undeployed position in accordance with an exemplary embodiment of the present disclosure;
**Fig. 4** is a cross sectional view of a housing of the lancet in accordance with an exemplary embodiment of the present disclosure;
**Fig. 5** is a cross sectional view of a housing of the lancet in accordance with an exemplary embodiment of the present disclosure;
**Fig. 6** is a cross sectional view of a housing of the lancet in accordance with an exemplary embodiment of the present disclosure;
**Fig. 7** is a bottom view of a housing of the lancet in accordance with an exemplary embodiment of the present disclosure;
**Fig. 8** is a cross sectional view of a cap of the lancet in accordance with an exemplary embodiment of the present disclosure;
**Fig. 9** depicts a side of an inner sleeve of the lancet in accordance with an exemplary embodiment of the present disclosure;
**Fig. 10** depicts another side of the inner sleeve of the lancet in accordance with an exemplary embodiment of the present disclosure;
**Fig. 11** is a cross sectional view of the inner sleeve of the lancet in accordance with an exemplary embodiment of the present disclosure;
**Fig. 12** depicts a side of a needle platform of the lancet in accordance with an exemplary embodiment of the present disclosure;
**Fig. 13** depicts another side of the needle platform in accordance with an exemplary embodiment of the present disclosure;
**Fig. 14** is a cross sectional view of a spring sleeve of the lancet in accordance with an exemplary embodiment of the present disclosure;
**Fig. 15** is a top view of a cover of a cartridge of the dermal patch system in accordance with an exemplary embodiment of the present disclosure;
**Fig. 16** is a cross sectional view of the cover in accordance with an exemplary embodiment of the present disclosure;
**Fig. 17** is a top view of a base of the cartridge of the dermal patch system in accordance with an exemplary embodiment of the present disclosure;
**Fig. 18** is a cross sectional view of a lancet receiving element of the base in accordance with an exemplary embodiment of the present disclosure;
**Fig. 19** is a top view of a base of the cartridge of the dermal patch system in accordance with an exemplary embodiment of the present disclosure;
**Fig. 20** is a cross sectional view of a lancet receiving element of the base in accordance with an exemplary embodiment of the present disclosure;
**Fig. 21** is a cross sectional view of a lancet receiving element of the base in accordance with an exemplary embodiment of the present disclosure;
**Fig. 22** is a cross sectional view of a lancet receiving element of the base in accordance with an exemplary embodiment of the present disclosure;
**Fig. 23** is another cross sectional view of a lancet receiving element of the base in accordance with an exemplary embodiment of the present disclosure;
**Fig. 24** is a cross sectional view of the base in accordance with an exemplary embodiment of the present disclosure;
**Fig. 25** schematically depicts a physiological sample well, a physiological sample channel, and a sample collection reservoir of the base in accordance with an exemplary embodiment of the present disclosure;
**Fig. 26** is a cross sectional view of the lancet engaged with the base, wherein the needle platform is in a released position in accordance with an exemplary embodiment of the present disclosure;
**Fig. 27** is another cross sectional view of the lancet engaged with the base, wherein the needle platform is in a released position in accordance with an exemplary embodiment of the present disclosure;

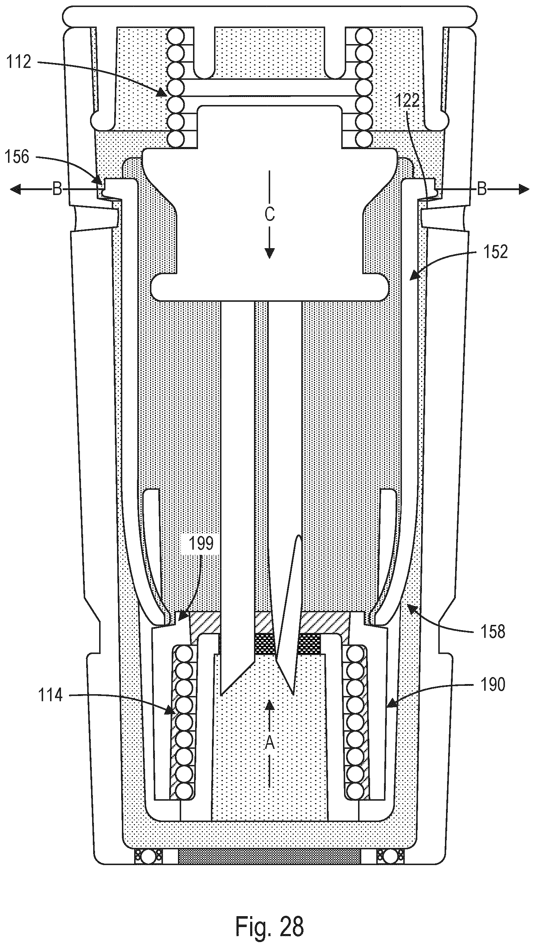

**Fig. 28** is a cross sectional view of the lancet, wherein the needle platform is in a released position **in** accordance with an exemplary embodiment of the present disclosure;
**Fig. 29** is a cross sectional view of the lancet transitioning to a deployed position in accordance with an exemplary embodiment of the present disclosure;
**Fig. 30** is a cross sectional view of the lancet engaged with the base, wherein the lancet is in a deployed position in accordance with an exemplary embodiment of the present disclosure;
**Fig. 31** is a cross sectional view of the lancet, wherein the lancet is in a deployed position in accordance with an exemplary embodiment of the present disclosure;
**Fig. 32** is a cross sectional view of the lancet engaged with the base, wherein the lancet is in a retracted position in accordance with an exemplary embodiment of the present disclosure;
**Fig. 33** is a cross sectional view of the lancet, wherein the lancet is in a retracted position in accordance with an exemplary embodiment of the present disclosure;
**Fig. 34** is a flowchart of a method for obtaining a physiological sample from a subject;
**Fig. 35** schematically depicts a computer system in accordance with an exemplary embodiment of the present disclosure; and
**Fig. 36** schematically depicts a cloud computing environment in accordance with an exemplary embodiment.

### DETAILED DESCRIPTION

The present disclosure generally relates to a dermal patch system (which may also be referred to as a "dermal patch") that may be utilized to collect and optionally store a physiological sample.

In various aspects, the present teachings address an unmet need for devices that allow efficient, easy and reliable extraction of biological samples from a subject.

In various embodiments, a dermal patch system is disclosed that allows collecting a physiological sample and storing the collected physiological sample, e.g., on a sample collection pad and/or a reservoir provided on the dermal patch system. Dermal patch systems disclosed herein may allow for the collection and analysis of a physiological sample in a variety of environments (e.g., at home, in the field, in a medical facility, etc.).

The term "about," as used herein, denotes a deviation of at most 10% relative to a numerical value. For example, about 100 µm means in the range of 90 µm-110 µm.

The term "substantially," as used herein, refers to a deviation, if any, of at most 10% from a complete state and/or condition.

The term "majority," as used herein, refers to more than 50% of an object or substance.

The term "subject" as used herein refers to a human subject or an animal subject (e.g., chicken, pig, cattle, dog, cat, etc.).

The term "transparent," as used herein, indicates that light can substantially pass through an object (e.g., a window) to allow visualization of a material disposed behind the object. For example, in some embodiments, a transparent object allows the passage of at least 70%, or at least 80%, or at least 90% of visible light therethrough.

The term "needle" as used herein, refers to a component with a pointed tip that is configured to pierce an outer surface of an element (e.g., skin of a subject) to provide a passageway through which a physiological fluid can be extracted.

The present disclosure generally relates to a device, which is herein also referred to as a dermal patch or a dermal patch system, for collecting a physiological sample (e.g., bodily fluids such as blood, interstitial fluids, etc.) from a subject. In some embodiments discussed below, such a dermal patch system can include a cartridge that can be affixed to a subject's skin (e.g., via an adhesive layer) and a separate lancet that can be engaged with the cartridge to puncture the skin, thereby providing a passageway for extracting the physiological sample. As discussed in more detail below, the lancet can include a housing in which at least one needle that is configured for puncturing the skin is disposed. The lancet can be transitioned between at least three states. In a first state (which may be referred to as an undeployed or a locked state) the lancet retains the needle(s) within the lancet housing when the lancet is not engaged with the cartridge. In a second state (which may be referred to as a deployed, an unlocked state, or a released state) the lancet allows the needle(s) to be deployed to allow the needle(s) to exit the housing, so as to be available for puncturing the skin in response to engagement of the lancet with the cartridge. In a third state (which may be referred to as a retracted state), subsequent to the deployment of the needle(s), the lancet automatically moves the needle(s) from the deployed position back into the lancet housing. In other words, the engagement of the lancet with the cartridge automatically transitions the needle(s) from the locked state to the unlocked state, and to the retracted state.

In some embodiments, as discussed in more detail below, the lancet includes an inner sleeve with a plurality of deformable tabs and a spring sleeve. In a locked state, the deformable tabs can apply a pressure to the spring sleeve to maintain the spring sleeve in a first position in which the spring sleeve compresses the lower spring into a compressed state and prevents the lower spring from expanding.

When the lancet is engaged with the cartridge, the engagement of the lancet with the cartridge causes the inner sleeve to move from a first position to a second position. In the first position, the inner sleeve retains a needle platform that supports the needles in an undeployed position. The lancet also includes an upper spring that is coupled to the needle platform. In the undeployed position, the needle platform compresses the upper spring and prevents the upper spring form expanding. In the second position, the inner sleeve moves vertically upward, which releases the needle platform thereby allowing the needle platform to move to a deployed position. When released, the upper spring is allowed to expand thereby applying a force to the needle platform, which causes the movement of the needle platform such that the needles extend beyond the housing of the lancet and become available for puncturing the skin.

Furthermore, this force causes the needles (which are coupled to the needle platform) to travel with sufficient velocity to puncture the skin of a subject. As the needle platform travels to the deployed position, the needle platform contacts the deformable tabs and causes the tabs to break or flex, e.g., to be deflected away from the lancet's longitudinal axis. When broken (or deflected), the tabs no longer contact the spring sleeve, or apply a lower force onto the spring sleeve, thereby allowing the lower spring to expand and move the spring sleeve vertically upward from a first position to a second position, which in turn moves the needle spring vertically upward such that the needles are retracted into the lancet's housing.

In this manner, the lancet remains safe before it is engaged with the cartridge as the needles in the lancet cannot be deployed when the lancet is not engaged with the cartridge. Furthermore, in this manner, the lancet remains safe after drawing a physiological sample as the needles automatically retract back into the lancet's housing after being deployed.

The cartridge can include a biometric sensor (e.g., a fingerprint scanner, iris scanner, etc.) and a computer system (e.g., a system on a chip "SOC") that is in communication with the biometric sensor. The biometric sensor is configured to obtain biometric data (e.g., fingerprint data, iris data) from a user of the dermal patch (e.g., a subject from whom a physiological sample is extracted, another user attaching the dermal patch to the subject, a medical professional, etc.). The biometric sensor is further configured to transmit the biometric data to the computer system. The computer system is configured to store the received biometric data in a memory. In this embodiment, the cartridge includes a power supply (e.g., a battery, a charged capacitor, etc.) that is in electrical communication with the biometric sensor and the computer system via an open circuit. The lancet and the cartridge each include electrical contacts that can engage with one another, e.g., automatically upon engagement of the lancet with the cartridge, to complete the circuit thereby allowing the power supply to power the biometric sensor and the computer system. That is, the biometric sensor can obtain biometric data of a subject only when the lancet is inserted into the cartridge attached to the subject's skin.

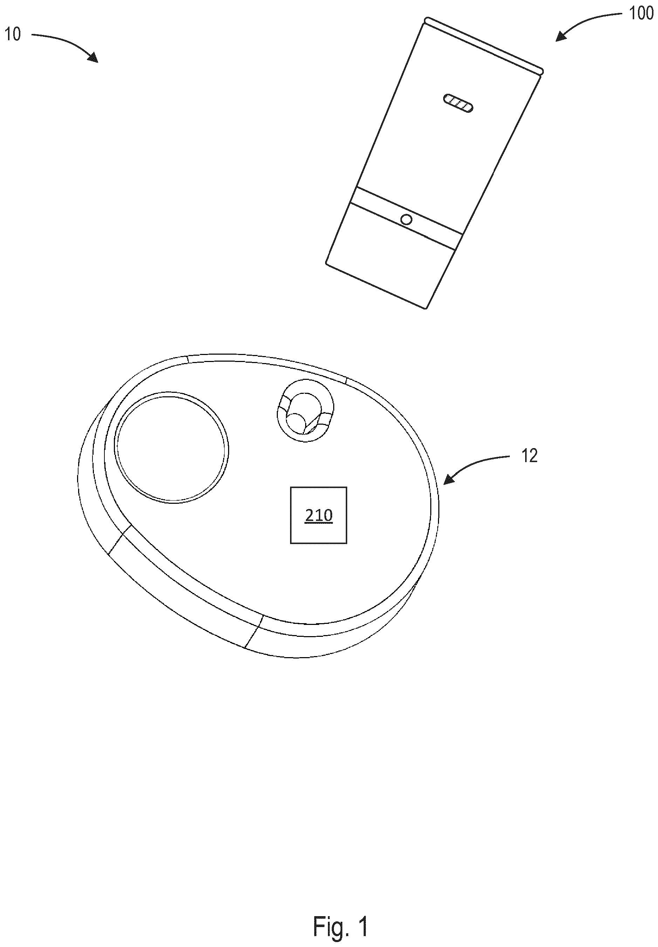

Referring now to **Fig. 1****,** a dermal patch system **10** is shown in accordance with an exemplary embodiment. In this embodiment, the dermal patch system **10** includes a cartridge **12** that can be affixed to a subject's skin and a lancet **100** that can be engaged with the cartridge **12.**

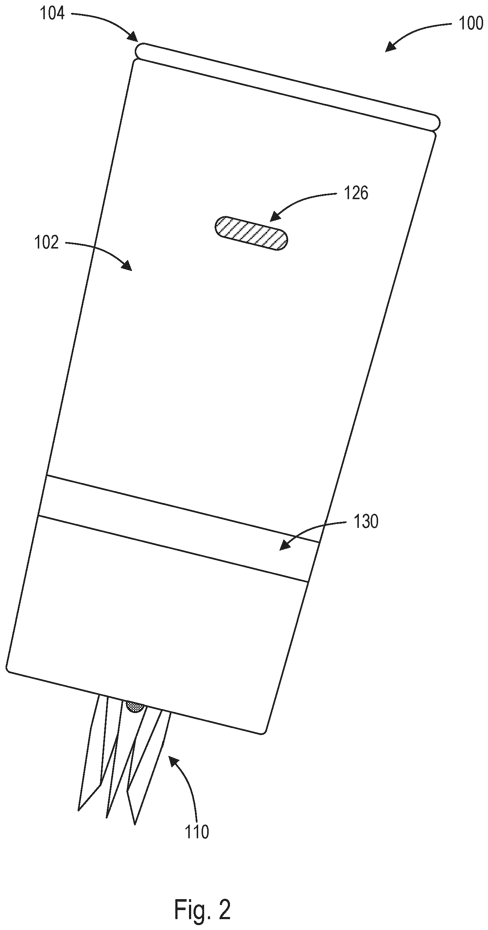

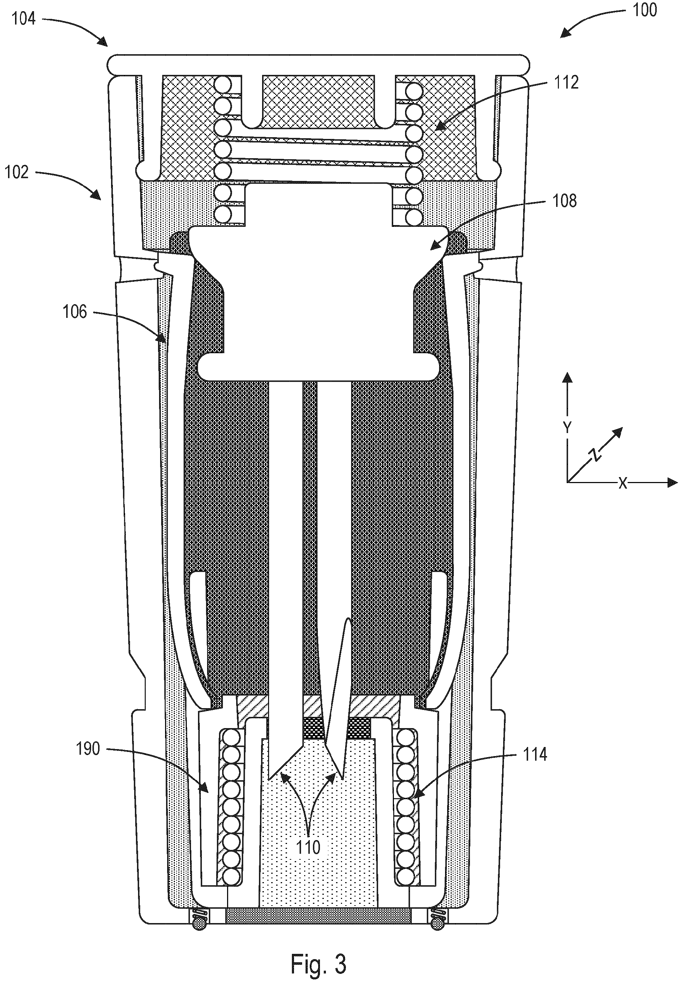

Referring to **Figs. 2** **and** **3****,** a cross sectional view of the lancet **100** is shown in accordance with an exemplary embodiment. The lancet **100** includes a housing **102** and a cap **104** that is coupled to the housing **102.** The lancet extends longitudinally along an X-axis **(****Fig. 3****)** between a proximal end defined by the cap **104** and a distal end defined by an end of the housing **102** that is opposite the cap **104.**

The housing **102** and the cap **104** define an inner volume of the lancet **100** in which various components of the lancet **100** are disposed. The lancet **100** further includes an inner sleeve **106** and a needle platform **108** that are disposed within the inner volume of the lancet **100.** The needle platform **108** supports a plurality of needles **110,** specifically three needles **110** in this embodiment.

While needle platform **108** is depicted as supporting three needles **110 (****Fig. 2****),** in other embodiments, the needle platform **108** can support more or less needles **110** (e.g., 1, 2, 5, needles etc.). However, it has been discovered that the use of three needles is particularly advantageous in drawing blood from a subject. Further, in this embodiment, the needles have slanted ends with the bottom ends of the needles facing away from one another.

The lancet **100** further includes an injection spring (also referred to as an upper spring) **112** and a retraction spring (also referred to as a lower spring) **114** that can move the needle platform **108** in a manner discussed in more detail below.

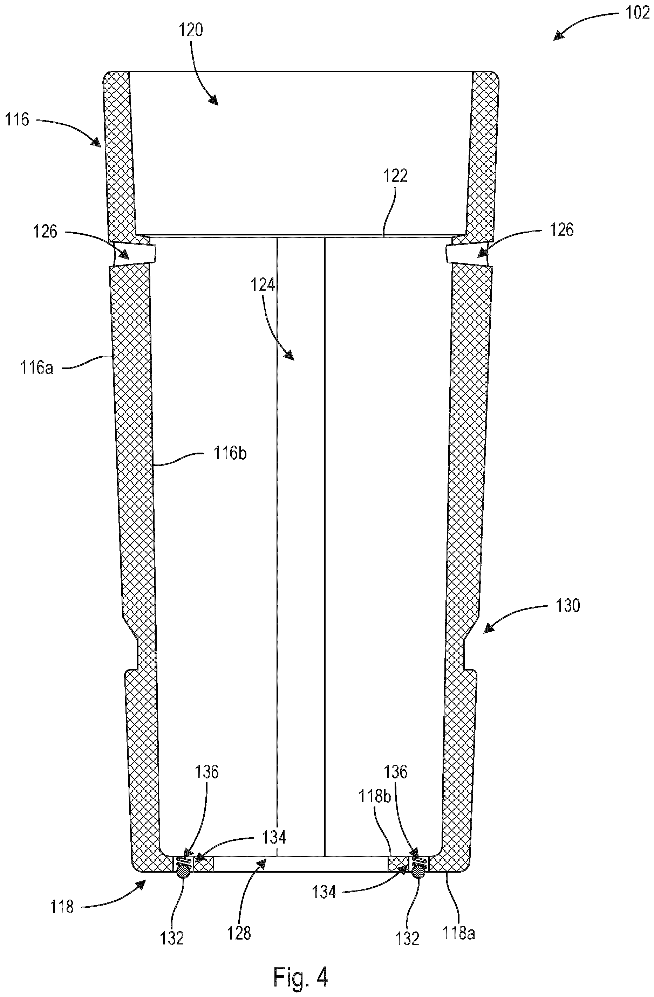

With particular reference to **Figs. 4-8****,** the housing **102** includes a side wall **116** and a bottom wall **118.** The side wall **116** includes an outer surface **116a** and an opposed inner surface **116b.** The bottom wall **118** includes an outer surface **118a** and an opposed inner surface **118b.** The side wall **116** extends substantially vertically from the bottom wall **118.** In this embodiment, the side wall **116** is substantially cylindrical in shape and the bottom wall **118** has a substantially circular cross-section. The side wall **116** and the bottom wall **118** are generally symmetric about a longitudinal axis (Y-axis) of the lancet **100.**

The inner surface **116b** of the side wall **116** and the inner surface **118b** of the bottom wall **118** define an inner volume **120.** The side wall **116** is tapered such that its inner diameter increases in a direction from its distal end to its proximal end such that the lancet housing exhibits a wider upper portion relative to its lower portion. Further, the inner surface **118b** of the upper portion defines a ledge **122.**

The inner surface **116b** also defines a first and second longitudinal groove **124** that are opposed from one another (only one of which is shown in the cross section in **Fig. 4****).** The grooves **124** extend between the ledge **122** and the inner surface **118b** of the bottom wall **118.**

The side wall **116** defines openings **126** that extend through the side wall **116.** That is, the openings **126** extend between the outer surface **116a** and the inner surface **116b** of the side wall **116.** The bottom wall **118** defines an aperture **128** that extends through the bottom wall **118.** That is, the aperture **128** extends between the outer surface **118a** and the inner surface **118b** of the bottom wall **118.** As will be discussed in further detail herein, when the lancet **100** is activated via engagement with the cartridge **12,** the needles of the lancet **100** extend through the aperture **128** for puncturing a subject's skin to draw a physiological sample. The outer surface **116b** of the side wall **116** further defines an outer groove **130.** As will be discussed in further detail herein, a portion of the cartridge **12** engages with the outer groove **130** to couple the lancet **100** to the cartridge **12.**

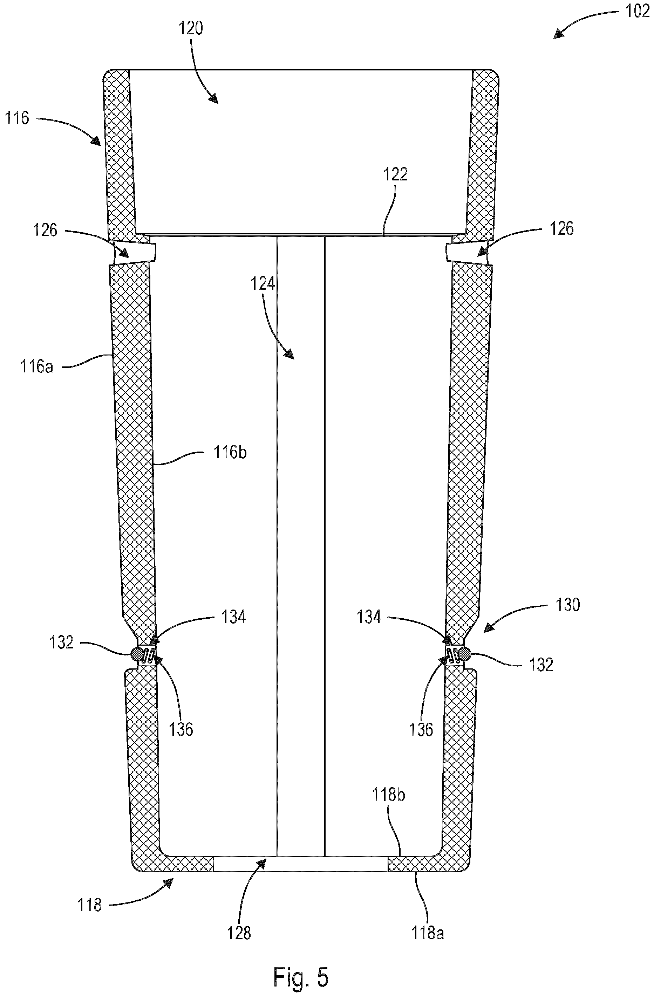

The housing **102** further includes a plurality of electrical contacts **132** are in electrical communication with one another and are formed of a material that is suitable for conducting electricity (e.g., copper, iron, gold, aluminum, etc.). In this embodiment, as depicted in **Figs. 4** and **5****,** the electrical contacts **132** are in the form of pogo style connectors and have a spherical shape. In one embodiment **(****Fig. 4****),** the bottom wall **118** includes contact receptacles **134** in each of which one of the electrical contacts **132** and a respective spring **136** can be housed.

When the lancet **100** is not engaged with the cartridge **12** the springs **136** are in a relaxed state and push the contacts **132** partially beyond the bottom wall **118.** While **Fig. 4** depicts the bottom wall **118** as including the electrical contacts **132,** the contact receptacles **134,** and the springs **136,** these elements may be disposed elsewhere on the housing **102.** For example, in another embodiment **(****Fig. 5****),** the groove **124** of the side wall **116** includes the contact receptacles **134**for housing the electrical contacts **132** and the respective springs **136.** In this embodiment, when the lancet **100** is not engaged with the cartridge **12** the springs **136** are in a relaxed state and push the contacts **132** partially beyond the side wall **116.** While the electrical contacts **132** are depicted as pogo style connectors, in other embodiments, the electrical contacts **132** may take other forms (e.g., electrical contact pads).

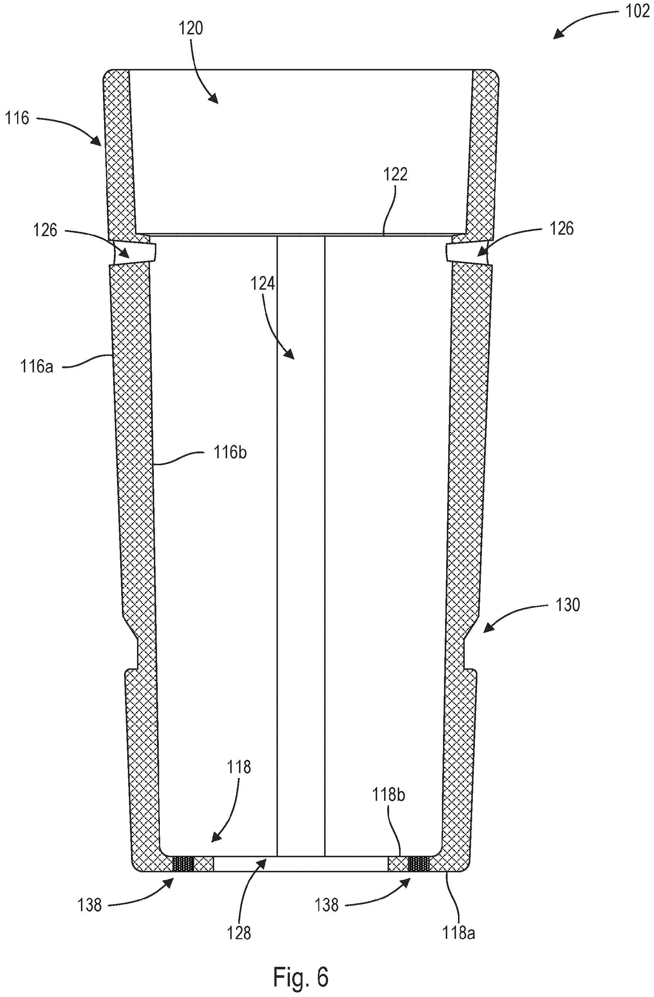

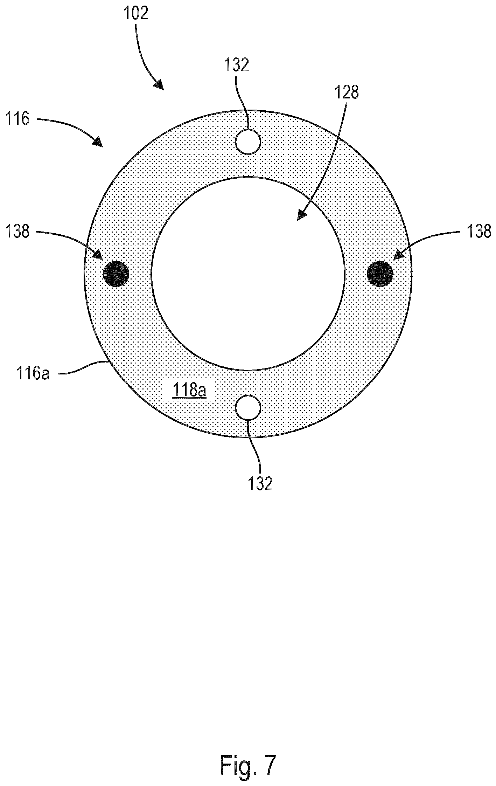

The bottom wall **118** further includes a plurality of openings **138 (****Figs. 6** **and** **7****)** that extends through bottom wall **118.** That is, the openings **138** extend between the outer surface **118a** and the inner surface **118b** of the bottom wall **118.** As will be discussed in further detail herein, the openings **138** aid in properly aligning the lancet **100** with the cartridge **12.**

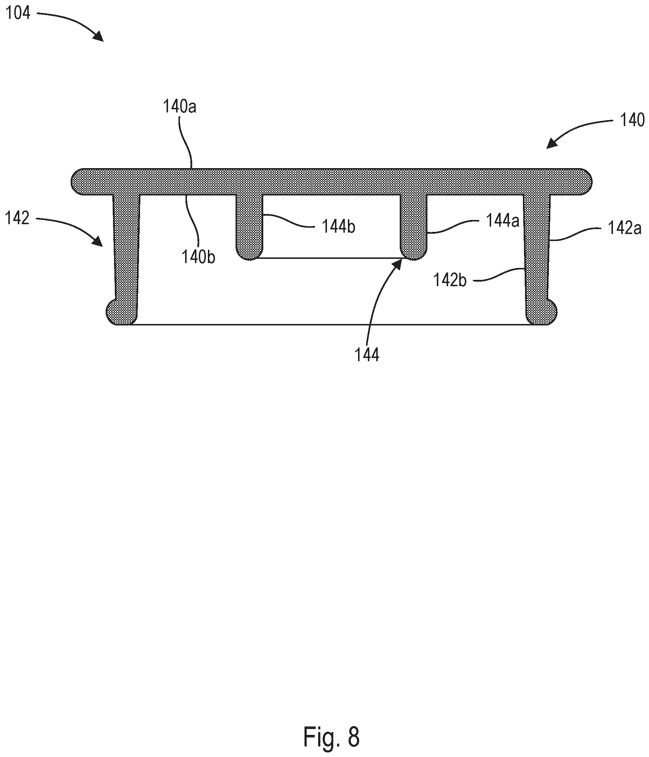

Referring now to **Fig. 8****,** the inner cap **104** is shown in accordance with an exemplary embodiment. The cap **104** includes a top wall **140** with an outer surface **140a** and an opposed inner surface **140b.** The cap **104** also includes a side wall **142** with an outer surface **142a** and an opposed inner surface **142b.** The top wall **140** extends substantially horizontally from and perpendicular to the side wall **142.** The side wall **142** extends substantially vertically from and perpendicular to the top wall **140.** The top wall **140** and the side wall **142** have generally circular cross-sectional profiles and are concentric with one another. The cap **104** also includes an inner cylinder **144** with an outer surface **144a** and an opposed inner surface **144b.** The inner cylinder **144** extends substantially vertically from and perpendicular to the top wall **140.** The inner cylinder **144** is generally concentric with the top wall **140** and the side wall **142.** The inner cylinder **144** extends substantially vertically from and perpendicular to the top wall **140.**

When the cap **104** is coupled to the housing **102** the side wall **142** extends into the inner volume **120** of the housing **102** and at least a portion of the side wall **142** contacts the inner surface **116b** of the side wall **116** such that the cap **104** couples to the housing **102** via an interference fit.

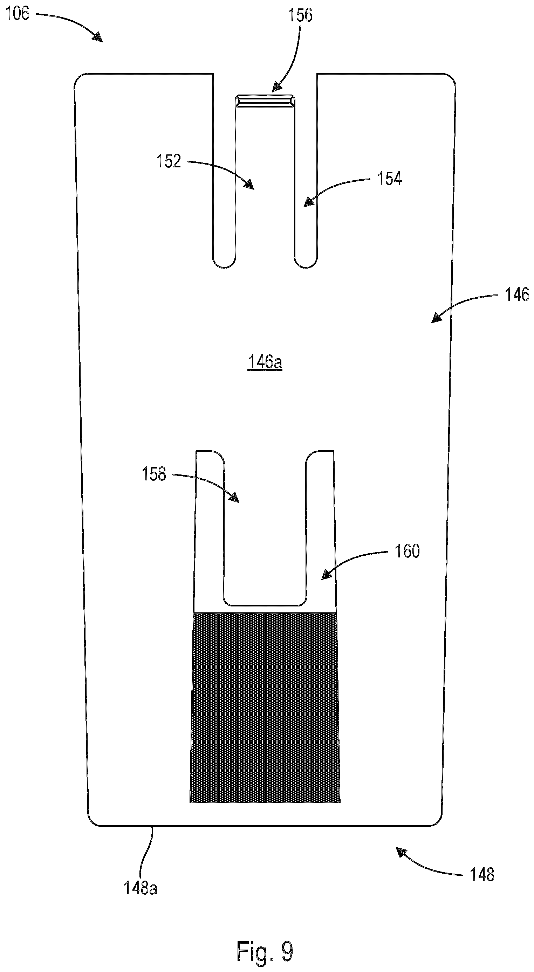

Referring now to **Figs. 9-11****,** the inner sleeve **106** is shown in accordance with an exemplary embodiment. The inner sleeve **106** includes a side wall **146** and a bottom wall **148.** The side wall **146** includes an outer surface **146a** and an opposed inner surface **146b.** The bottom wall **148** includes an outer surface **148a** and an opposed inner surface **148b.** The side wall **146** extends substantially vertically from the bottom wall **148.** The side wall **146** and the bottom wall **148** have generally circular cross-sectional profiles and are positioned concentrically relative to one another. The inner surface **146b** of the side wall **146** and the inner surface **148b** of the bottom wall **148** define an inner volume **150.**

The side wall **146** defines a first and second deformable extension **152** that extend within a gap **154** of the side wall **146.** The extensions **152** each include a hook **156.** As will be discussed in further detail herein, the hooks **156** of the extensions **152** can be received by the openings **126** of the housing **102** to place the lancet **100** in the undeployed position.

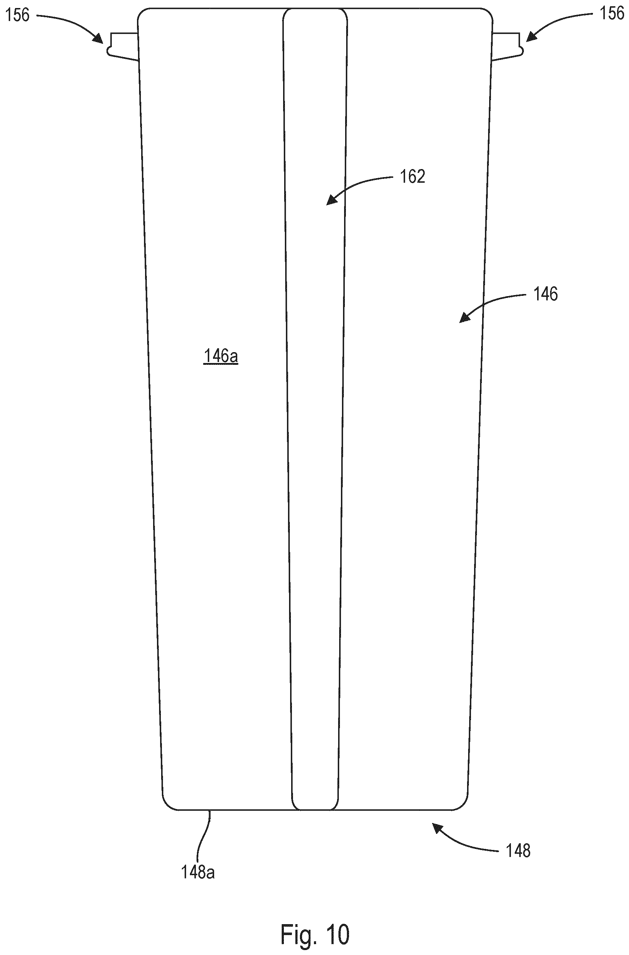

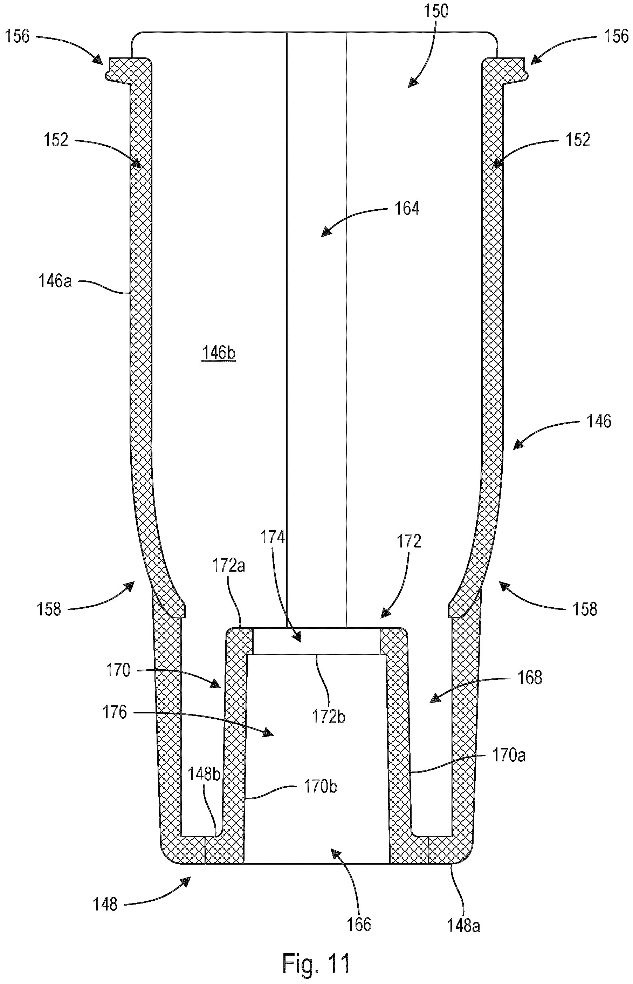

The side wall **146** further defines a first and second deformable tabs **158** that extend within an opening **160** of the side wall **146.** The side wall **146** also includes a first and second protrusions **162** (only one of which is shown in **Fig. 10****)** that extend substantially horizontally from and perpendicular to the outer surface **146a** of the side wall **146.** When the inner sleeve **106** is disposed within the housing **102,** the protrusions **162** extend within the grooves **124** of the housing **102.** When the inner sleeve **106** is moving between different positions (e.g., between an undeployed position to a deployed position), the grooves **124** and the protrusions **162** guide the vertical movement of the inner sleeve **106** while preventing a rotational or horizontal movement of the inner sleeve **106.** The inner surface **146b** of the side wall **146** further defines a first and second grooves **164** that are opposed from one another (only one of which is shown in **Fig. 11****).** The grooves **164** extend substantially vertically from and perpendicular to the inner surface **148b** of the bottom wall **148.**

The inner sleeve **106** includes an opening **166** that extends through the bottom wall **148.** That is, the opening **166** extends between the outer surface **148a** and the inner surface **148b** of the bottom wall **148.** The inner sleeve **106** also includes a post **168** that is cylindrical in shape and is generally concentric with the opening **166.** The post **168** includes a side wall **170** and a top wall **172.** The side wall **170** extends substantially vertically from and perpendicular to the inner surface **148b** of the bottom wall **148.** The side wall **170** includes an outer surface **170a** and an opposed inner surface **170b.** The top wall **172** extends substantially horizontally between the side wall **170** and includes a top surface **172a** and an opposed bottom surface **172b.** The top wall **172** includes an opening **174** that extends through the top wall **172.** That is, the opening **174** extends between the top surface **172a** and the bottom surface **172b** of the top wall **172.** The inner surface **170b** of the side wall **170** and the bottom surface **172b** of the top wall **172** define an inner volume **176** of the post **168.** As will be discussed in further detail herein, the needles **110** extend through the opening **174** of the top wall **172,** through the inner volume **176,** and through the opening **166** of the bottom wall **148** when in the deployed position.

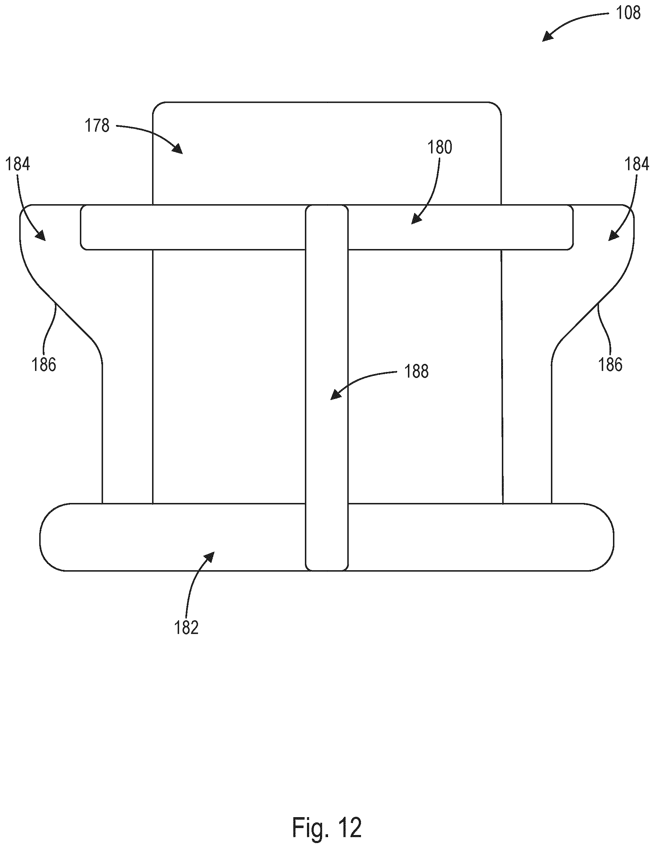

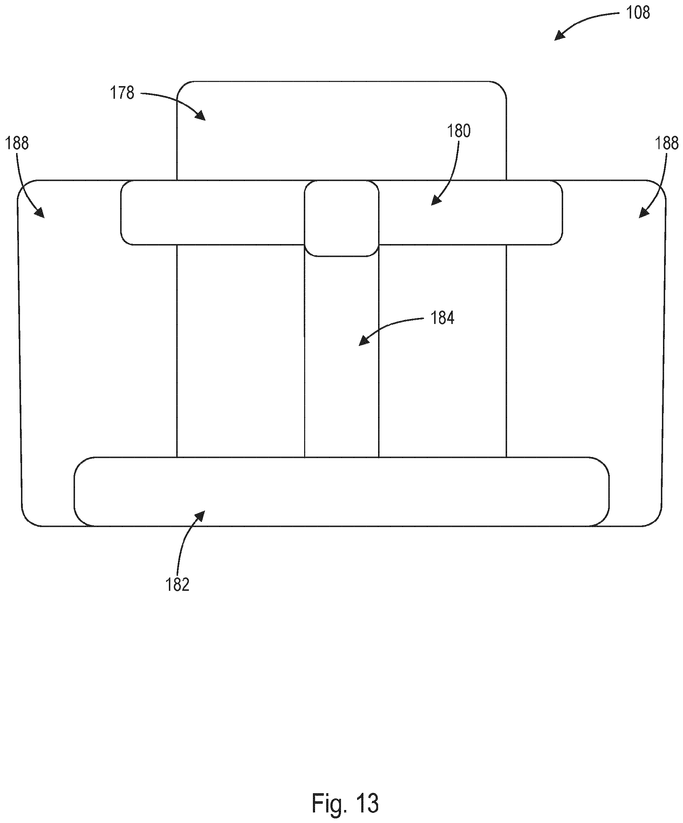

Referring now to **Figs. 12** **and** **13****,** the needle platform **108** is shown in accordance with an exemplary embodiment. The needle platform **108** includes a cylindrical body **178,** an upper circular extension **180** and a lower circular extension **182.** The upper circular extension **180** and the lower circular extension **182** extend substantially horizontally from and perpendicular to the cylindrical body **178.** As will be discussed in further detail herein, the upper circular extension **180** serves as a platform for the injection spring **112.** The needle platform **108** further includes a first and second curved extensions **184.** The curved extensions **184** extend substantially horizontally from and perpendicular to the cylindrical body **178** and extend substantially vertically between the upper circular extension **180** and the lower circular extension **182.** Each curved extension **184** includes an angled surface **186.** As will be discussed in further detail herein, when the needle platform is in the undeployed position, the angled surfaces **186** contact the hooks **156** of the inner sleeve **106,** which prevents the needle platform **108** from transitioning to the deployed position.

The needle platform **108** further includes first and second projections **188.** The first and second projections **188** are generally rectangular in shape, extend substantially horizontally from and perpendicular to the cylindrical body **178,** and extend substantially vertically between the upper circular extension **180** and the lower circular extension **182.** When the needle platform **108** is disposed within the inner sleeve **106,** the projections **188** extend into the grooves **164** which guides the vertical movement of the needle platform **108,** i.e., in a direction substantially parallel to the longitudinal axis of the lancet, while preventing a rotational or horizontal movement of the needle platform **108.**

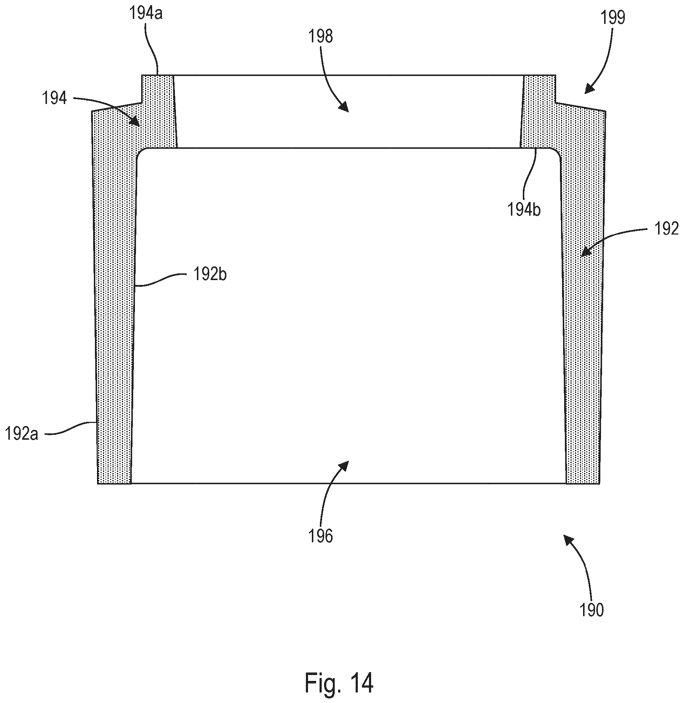

As depicted in **Fig. 3****,** the lancet **100** further includes a spring sleeve **190.** With particular reference to **Fig. 14****,** the spring sleeve **190** is shown in accordance with an exemplary embodiment. The spring sleeve **190** is cylindrical in shape and includes a side wall **192** and a top wall **194.** The side wall **192** includes an outer surface **192a** and an opposed inner surface **192b** and the top wall **194** includes a top surface **194a** and an opposed bottom surface **194b.** The inner surface **192b** of the side wall **192** and the bottom surface **194b** of the top wall **194** define an inner volume **196** of the spring sleeve **190.** The side wall **192** and the top wall **194** are shaped and dimensioned such that the spring sleeve **190** retains the retraction spring **114** within the inner volume **196.** The top wall **194** includes an opening **198** that extends therethrough. That is, the opening **198** extends between the top surface **194a** and the bottom surface **194b** of the top wall **194.** The opening **198** of the top wall **194** is in communication with the inner volume **196.** Since the inner volume **196** is open, the needles **110** may extend through spring sleeve **190** via the opening **198.** The outer surface **192a** defines a ledge **199** that extends circumferentially around the spring sleeve **190.** As will be discussed in further detail herein, when the lancet **100** is in the undeployed position, the tabs **158** contact the ledge **199.**

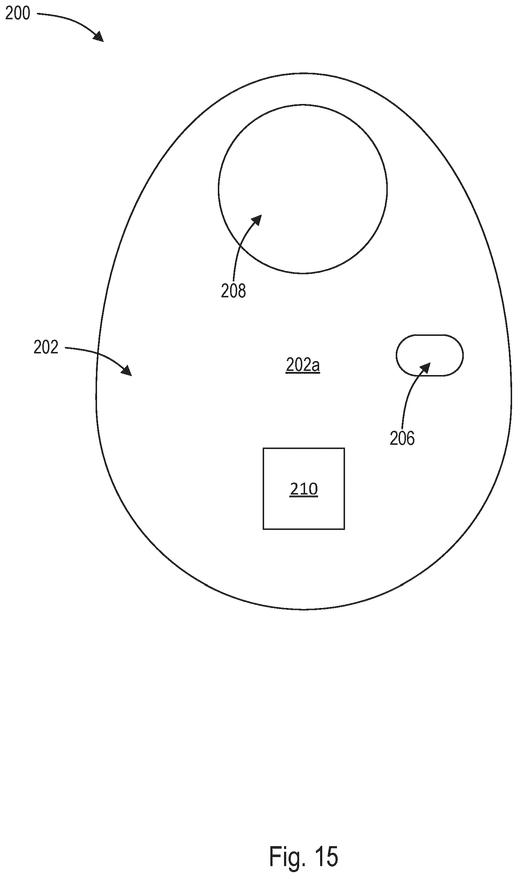

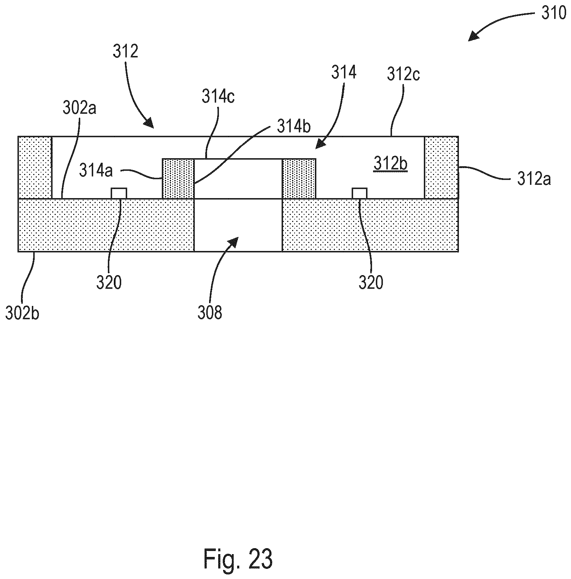

Referring now to **Figs. 15-25****,** the cartridge **12** is shown in accordance with an exemplary embodiment. The cartridge **12** is configured to attach to the skin of a subject via an adhesive layer (not shown) disposed on a bottom surface of the cartridge **12.** After use, the cartridge **12** may be removed by pulling it off the skin of the subject. The cartridge **12** includes a cover **200** and a base **300** that can couple to the cover **200.** In one embodiment, the cover **200** and the base **300** are formed as two separate components that are removably coupled to one another (e.g., via a snap fitting). In another embodiment, the cover **200** and the base **300** form an integral unitary cartridge **12.** The cover **200** and the base **300** may be coupled to one another via an adhesive, laser welding, etc.

The cartridge **12** may be formed using a variety of suitable materials including, but not limited to, polymeric materials (e.g., polyolefins, polyethylene terephthalate (PET), polyurethanes, polynorbornenes, polyethers, polyacrylates, polyamides (Polyether block amide also referred to as Pebax^{®}), polysiloxanes, polyether amides, polyether esters, trans-polyisoprenes, polymethyl methacrylates (PMMA), cross-linked trans-polyoctylenes, cross-linked polyethylenes, cross-linked polyisoprenes, cross-linked polycyclooctenes, inorganic-organic hybrid polymers, co-polymer blends with polyethylene and Kraton^{®}, styrene-butadiene co-polymers, urethane-butadiene co-polymers, polycaprolactone or oligo caprolactone co-polymers, polylactic acid (PLLA) or polylactide (PL/DLA) co-polymers, PLLA-polyglycolic acid (PGA) co-polymers, photocross linkable polymers, etc.). In some embodiments, some of the cover **200** may be formed of poly(dimethylsiloxane) (PDMS) to allow visibility of components disposed within the cartridge **12.**

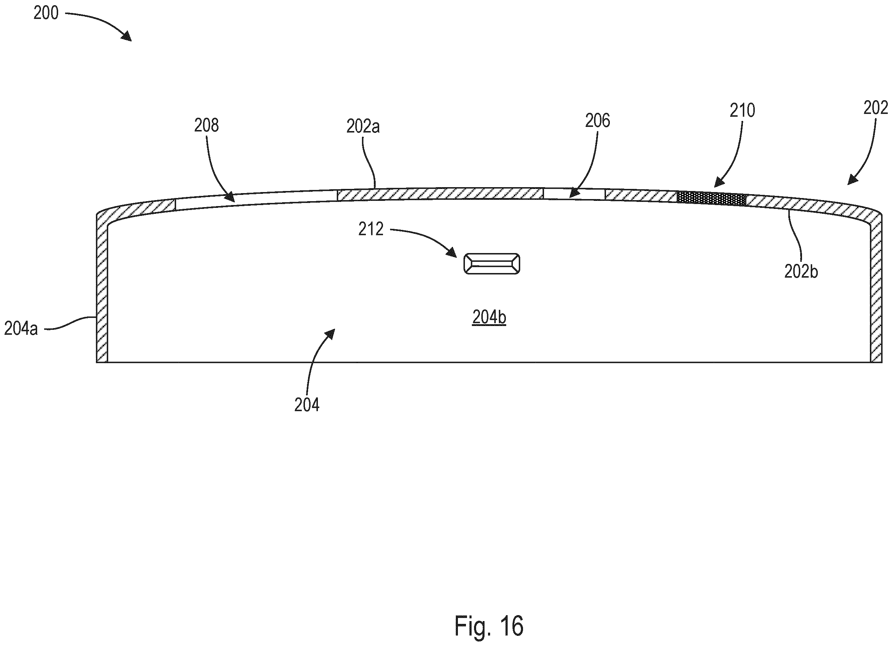

Referring now to **Figs. 15** **and** **16****,** the cover **200** is shown in accordance with an exemplary embodiment. The cover **200** includes a top wall **202** with an outer surface **202a** and an opposed inner surface **202b.** The cover **200** also includes a side wall **204** with an outer surface **204a** and an opposed inner surface **204b.** The top wall **202** extends substantially longitudinally from and perpendicular to the side wall **204.** The side wall **204** extends substantially vertically from and perpendicular to the top wall **202.**

The cover **200** includes a viewing aperture **206** that extends through the top wall **202.** That is, the viewing aperture **206** extends between the outer surface **202a** and the inner surface **202b** of the top wall **202.** In various embodiments, the viewing aperture **206** is covered by a transparent material, e.g., a transparent polymeric material, which allows viewing the components disposed within the cartridge **12.** The top wall **202** further includes a lancet aperture **208** that extends through the top wall **202.** That is, the lancet aperture **208** extends between the outer surface **202a** and the inner surface **202b** of the top wall **202.** The lancet aperture **208** is generally shaped and dimensioned to accept a portion of the lancet **100,** e.g., the aperture can be substantially circular. As will be discussed in further detail herein, a portion of the lancet **100** extends through the lancet aperture **208** to couple to the base **300.**

The cover **200** also includes a biometric sensor (e.g., a fingerprint scanner, iris scanner, etc.) **210** that is configured to obtain a fingerprint of a user of the dermal patch system **10.** While the biometric sensor **210** is disposed on the top wall **202** of the cover **200,** in other embodiments, the biometric sensor **210** may be located elsewhere (e.g., on the side wall **204).** The cover **200** further includes a plurality of locking members **212** that extend substantially horizontally from and perpendicular to the inner surface **204a** of the side wall **204.** As will be discussed in further detail herein, the locking members **212** aid in coupling the cover **200** to the base **300.** For the biometric sensor, a finger image sensor (FIS), or a contact image sensor (CIS), such as the M116-A8CIU sensor or the M116-A6C1P sensor made by CMOS Sensor Inc. in Cupertino, CA, can be used. Also, optical finger image sensors can be used as they can be more sensitive and durable than capacitive fingerprint sensors, e.g., 4x6mm sensing area. Optical finger image sensors generally include a light source and an optically clear cover, which is typically glass but a polymer or epoxy as substitute can be used. Alternatively, a capacitive fingerprint sensor that relies on the valleys and ridges of the finger could also be used. Also, flexible organic photodetectors can be used, as described in "Advances in Flexible Organic Photodetectors: Materials and Applications," Nanomaterials 2022, 12, 3775 (https://doi.org/10.3390/nano12213775), which is hereby incorporated by reference in its entirety. For further information on organic photodetectors (OPD's), please also see https://www.isorg.fr/, which also is hereby incorporated by reference in its entirety.

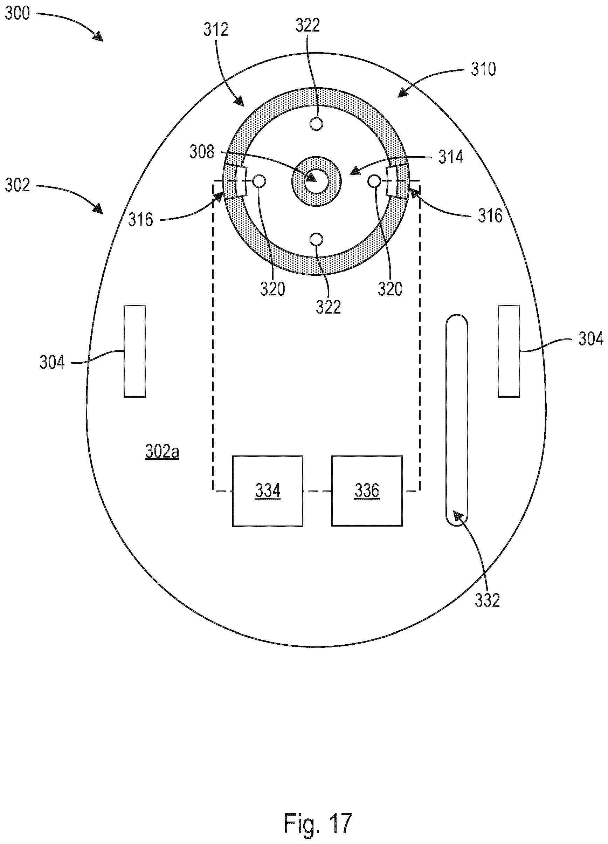

Referring now to **Figs. 17-25****,** the base **300** is shown in accordance with an exemplary embodiment. The base **300** includes a bottom wall **302** having a top surface **302a** and an opposed bottom surface **302b.** The bottom wall **302** has a similar shape and dimension as the side wall **204** of the cover **200** such that the cover **200** and the base **300** are flush with one another when the cover **200** is coupled to the base **300.** Furthermore, when the cover **200** is coupled to the base **300,** the side wall **204** contacts the bottom wall **302.**

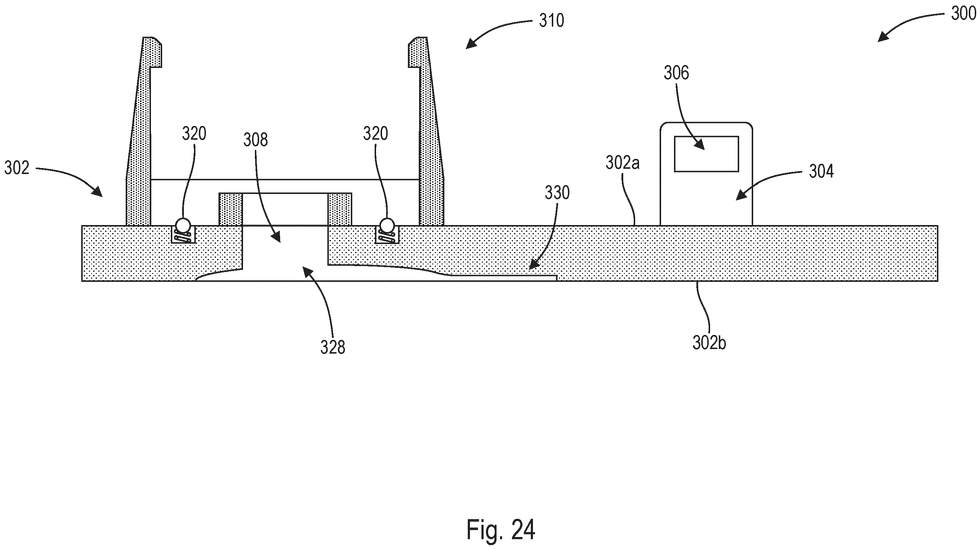

The base **300** includes a plurality of extensions **304** that extend substantially vertically from and perpendicular to the top surface **302a** of the bottom wall **302.** Each of the extensions **304** define a gap **306 (****Fig. 24**). The gap **306,** and therefore the extensions **304,** are shaped and dimensioned to accept a locking member **212.** The locking members **212** extend through the gaps **306** to couple the cover **200** to the base **300.**

The base **300** further includes a needle aperture **308** that is generally circular in shape. The needle aperture **308** extends through the bottom wall **302.** That is, the needle aperture **308** extends between the top surface **302a** and the bottom surface **302b** of the bottom wall **302.** As will be discussed in further detail herein, when the cover **200** is coupled to the base **300** and when the cartridge **12** is adhered to a subject, the needle aperture **308** allows the needles **110** of the lancet **100** to extend through the bottom wall **302** to puncture the subject's skin.

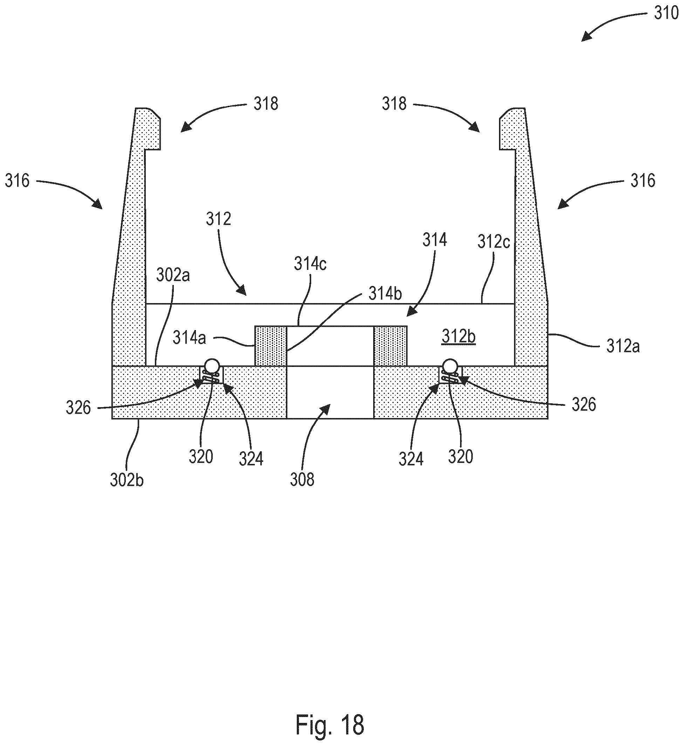

The base **300** also includes lancet receiving element **310** that is shaped and dimensioned to accept a distal end of the lancet **100.** With particular reference to **Figs. 18** and **20-****23,** the lancet receiving element **310** includes an outer circular projection **312** and an inner circular projection **314,** each extending substantially vertically from and perpendicular to the top surface **302a** of the bottom wall **302.** The outer circular projection **312** includes an outer surface **312a,** an opposed inner surface **312b,** and a top surface **312c** that extends between the outer surface **312a** and the inner surface **312b.** The top surface **312c** extends between the outer surface **312a** and the inner surface **312b** and is substantially perpendicular to those surfaces. The outer surface **312a** and the inner surface **312b** extend substantially vertically from and perpendicular to the top surface **302a** of the bottom wall **302.** The outer circular projection **312** is shaped to accept the lancet **100.**

The inner circular projection **314** is disposed around the needle aperture **308** and includes an outer surface **314a,** an opposed inner surface **314b,** and a top surface **314c** that extends between the outer surface **314a** and the inner surface **314b.** The top surface **314c** extends between the outer surface **314a** and the inner surface **314b** and is substantially perpendicular to those surfaces. The outer surface **314a** and the inner surface **314b** extend substantially vertically from and perpendicular to the top surface **302a** of the bottom wall **302.** The outer circular projection **312** and the inner circular projection **314** circular projection are substantially concentric with one another.

As will be discussed in further detail herein, when the lancet **100** is engaged with the cartridge **12,** the top surface **314c** of the inner circular projection contacts a portion of the lancet **100** which causes the lancet **100** to transition the needles **110** from the undeployed position to the deployed position.

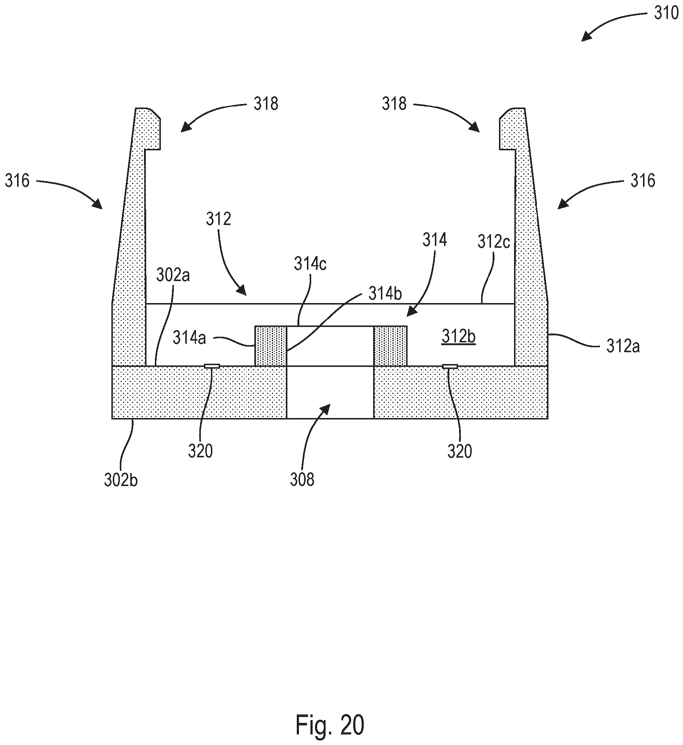

Withe continued reference to **Figs. 18** and **20****-22,** the lancet receiving element **310** further includes a plurality of locking members **316** that extend substantially vertically from and perpendicular to the top surface **312c** of the outer circular projection **312.** While **Figs. 20-22** depict the lancet receiving element **310** as including two locking members **316,** it is understood that, in other embodiments, the lancet receiving element **310** may include more or less locking members **316** (e.g., 1, 3, 5, etc.). Each locking member **316** includes a hook **318** that extends inwardly from a top of a locking member **316** towards the inner circular projection **314.** As will be discussed in further detail herein, the hooks **318** of the locking members **316** couple to the lancet **100** to retain the lancet **100** within the base **300.**

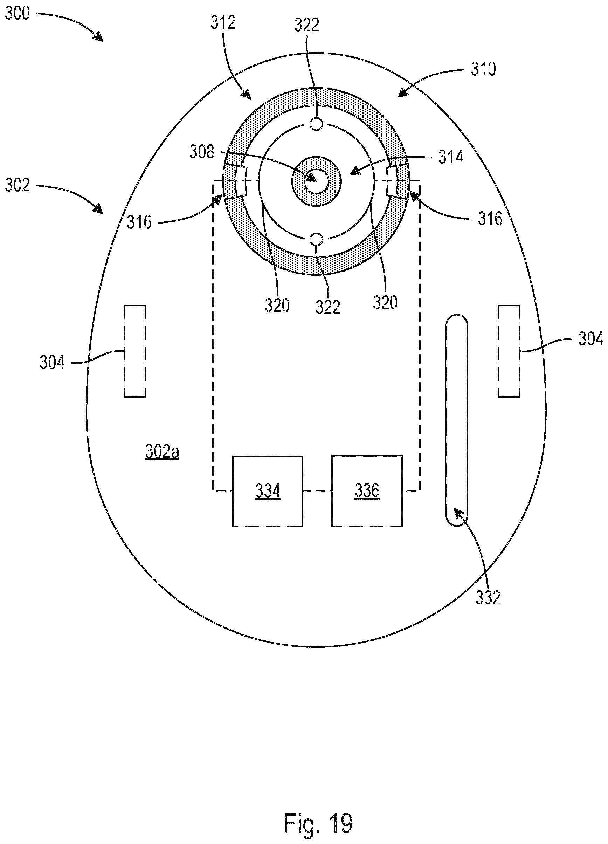

The lancet receiving element **310** further includes a plurality of electrical contacts **320** and a plurality of extensions **322.** The extensions **322** are substantially cylindrical in shape and extend substantially from and perpendicular to the top surface **302a** of the bottom wall **302.** The electrical contacts **320** are in electrical communication with one another and are formed of a material that is suitable for conducting electricity (e.g., copper, iron, gold, aluminum, etc.). In the embodiment depicted in **Figs. 17** and **18****,** the electrical contacts **320** are in the form of pogo style connectors. In this embodiment, the bottom wall **302** includes contact receptacles **324** each of which houses one of the electrical contacts **320** and a respective spring **326.** When the lancet **100** is not engaged with the cartridge **12** the springs **326** are in a relaxed state and push the contacts **320** partially beyond the bottom wall **302.** In another embodiment **(****Figs. 19** and **20****),** the electrical contacts **320** may take the form of an electrically conductive pad that extends substantially vertically from and perpendicular to the top surface **302a** of the bottom wall **302.** In these embodiments, the contact receptacles **324** and the springs **326** are omitted.

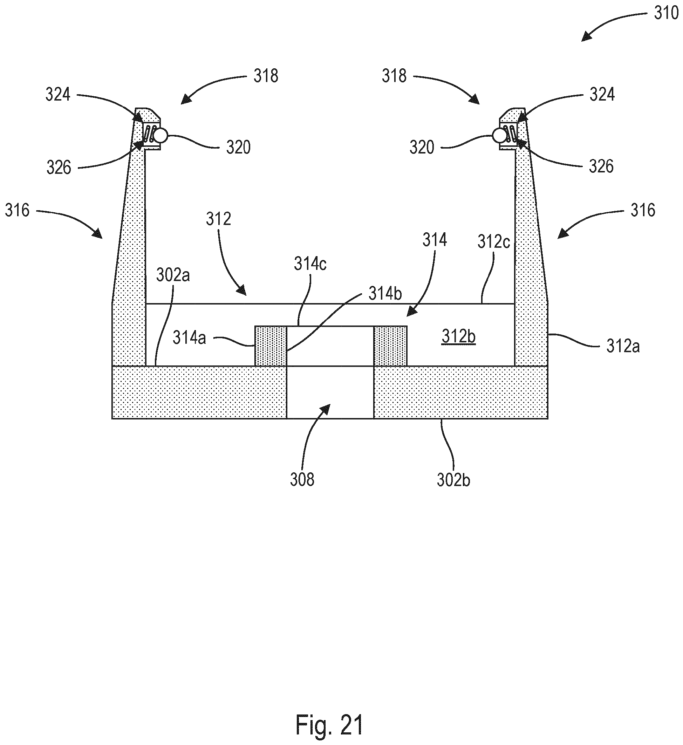

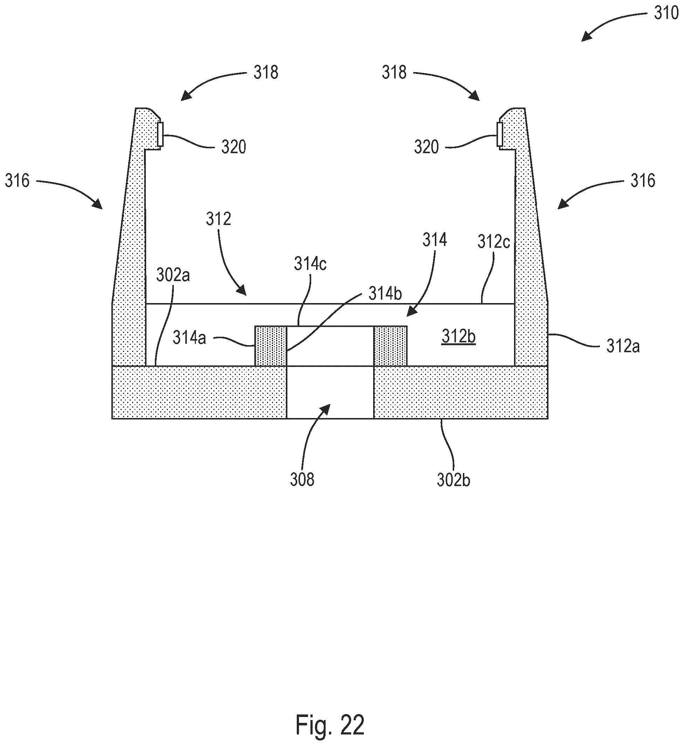

While **Figs. 17-20****,** depict the electrical contacts **320** as located on the bottom wall **302,** in another embodiment, when electrical contacts **132** of the lancet **100** are disposed on the side wall **116,** the hooks **318** of the locking members **316** can include the electrical contacts **320.** In one embodiment **(****Fig. 21****),** where the electrical contacts **320** are in the form of pogo style connectors, the hooks **318** can include the contact receptacles **324** and the springs **326.** In another embodiment **(****Fig. 22****),** the electrical contacts **320** disposed on the hooks **318** can take the form of an electrical pad and, in this embodiment, the contact receptacles **324** and the springs **326** are omitted.

With particular reference to **Fig. 24****,** the base **300** includes a physiological sample well **328** and a physiological sample channel **330** that extends radially from and is in open communication with the physiological sample well **328.** As will be discussed in further detail herein, the physiological sample channel **330** is a fluidic channel that is configured to carry a physiological sample extracted from a subject. The physiological sample well **328** and a portion of the physiological sample channel **330** are open with respect to the bottom surface **302b** of the bottom wall **302.** Stated another way, the physiological sample well **328** and a portion of the physiological sample channel **330** do not include a bottom surface. The physiological sample well **328** is in open communication with the needle aperture **308.** As will be discussed in further detail herein, when drawing a physiological sample, the needles **110** of the lancet **100** extend through the needle aperture **310** and through the physiological sample well **328** to pierce the skin of the subject. The physiological sample channel **330** extends from the physiological sample well **328** and through the bottom wall **302** of the base **300.**

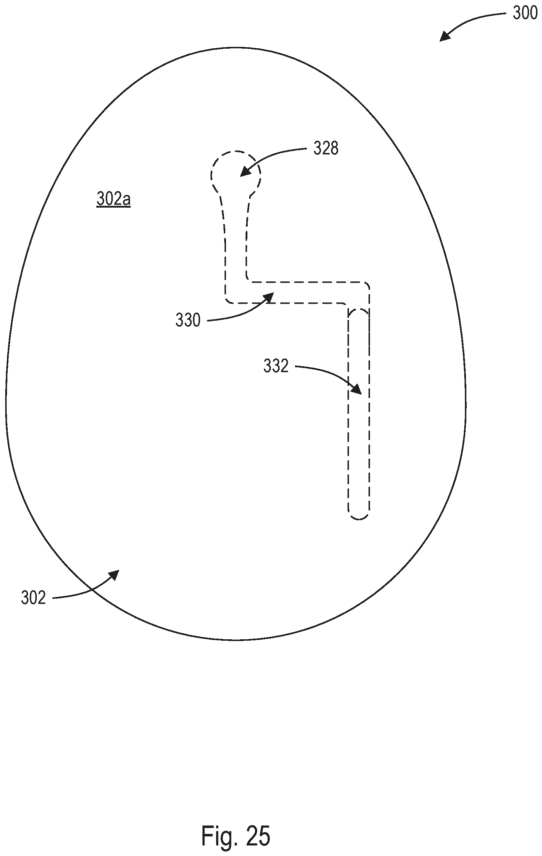

The bottom wall **302** of the base **300** further includes a sample collection reservoir **332 (****Fig. 25****)** that is configured to receive and store a drawn physiological sample. The sample collection reservoir **332** is in open communication with the physiological sample channel **330.** Accordingly, the physiological sample channel **330** carries a drawn physiological sample from the physiological sample well **328** to the sample collection reservoir **332** via capillary action. The sample collection reservoir **332** is disposed below the viewing aperture **206.** In one embodiment, the collection reservoir **332** is covered by a transparent material which allows a user of the dermal patch system **10** to view the sample collection reservoir **332** via the viewing aperture **206.** In some embodiments, a sample collection pad (e.g., a CF12 collection pad) is disposed within the sample collection reservoir **332** and absorbs the drawn physiological sample.

The base **300** includes an adhesive layer (not shown) disposed on the bottom surface **302b** of the bottom wall **302.** The cartridge **12** can be attached to the skin of a subject via the adhesive layer. The cartridge **12** may be attached anywhere on the subject's skin that is capable of supporting the cartridge **12** (e.g., on a leg, arm, etc. of the subject). The adhesive layer includes an opening that surrounds the physiological sample well **328** and through which the needles **110** can extend to puncture the subject's skin. The adhesive layer covers and therefore seals the open portion of the physiological sample channel.

The base **300** further includes a computer system (e.g., a SOC) **334** and a power supply (e.g., a battery, charged capacitor, etc.) **336.** The computer system **334** is connected to and in communication with the biometric sensor **210.** The power supply **336** is in electrical communication with the biometric sensor **210,** the computer system **334,** and the electrical contacts **320** such that in absence of engagement of the lancet with the cartridge, an open circuit is established that prevents the application of power by the power supply **336** to the biometric sensor **210** and the computer system **334.** As will be discussed in further detail herein, when the lancet **100** is engaged with the cartridge **12** the circuit is closed which allows the power supply **336** to power the biometric sensor **210** and the computer system **334.** As will be discussed in further detail herein, when powered, the biometric sensor **210** obtains biometric data, such as fingerprint data, from a user of the dermal patch system **10.**

Before the lancet **100** engages with the cartridge **12,** the needles **110** are in an undeployed position **(****Fig. 3****).** In the undeployed position, the hooks **156** of the inner sleeve **106** extend through the openings **126** of the housing **102,** which retains the lancet **100** in the undeployed position and prevents the lancet **100** from moving to the deployed position. In this position, the extensions **152** are compressed inward toward the center of the lancet **100,** which allows the needle platform **108** to rest upon the inner sleeve **106** thereby preventing the needle platform from moving vertically downward and into the deployed position. Specifically, the angled surface **186** of the extensions **184** of the needle platform **108** rests upon the extensions **152,** thereby inhibiting the movement of the needle platform.

Furthermore, the injection spring **112** extends around the body **178** of the needle platform **108** and the outer surface **144a** of the inner cylinder **144.** The injection spring **112** extends substantially vertically between the inner surface **140b** of the top wall **140** of the cap **104** and the upper circular extension **180** of the needle platform **108.** In the undeployed position, the injection spring **112** is compressed between the top wall **140** of the cap **104** and the upper circular extension **180** of the needle platform **108.** The retraction spring **114** extends around the post **168** of the inner sleeve **106.** The retraction spring **114** extends substantially vertically between the bottom wall **148** of the inner sleeve **106** and the top wall **194** of the spring sleeve **190.** That is, the retraction spring **114** extends substantially vertically between the inner surface **148b** of the bottom wall **148** of the inner sleeve **106** and the inner surface **194b** of the top wall **194** of the spring sleeve **190.** When the lancet **100** is in the undeployed position, the retraction spring **114** is compressed between the bottom wall **148** of the inner sleeve **106** and the top wall **194** of the spring sleeve **190.** Furthermore, in the undeployed position, the deformable tabs **158** contact the ledge **199** of the spring sleeve **190** so as to prevent the retraction spring from expanding and moving the spring sleeve **190.**

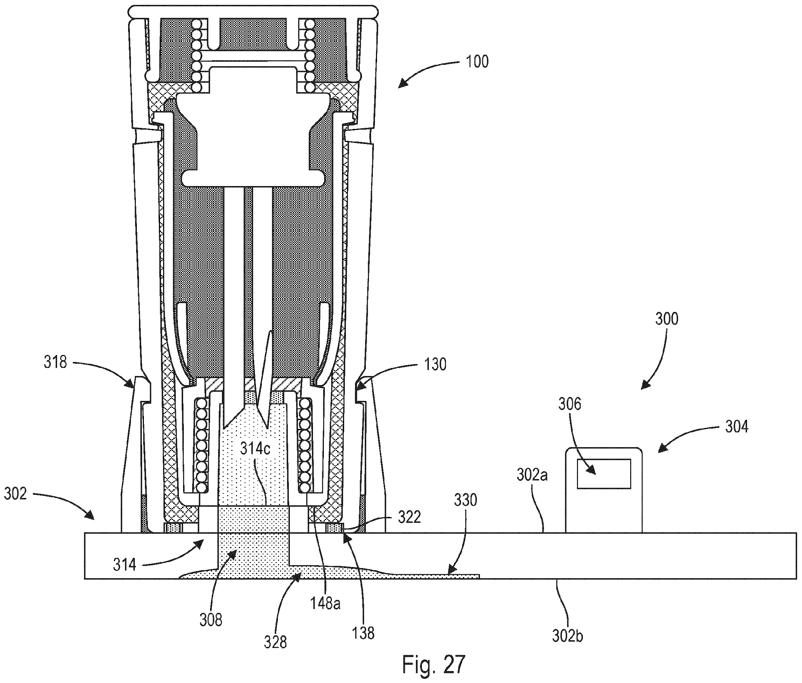

The lancet **100** can be engaged with the cartridge **12** by pushing the lancet **100** into the cartridge **12.** In order to transition the needles **110** to the deployed position and power the biometric sensor **210** and the computer system **334,** the lancet **100** must be properly aligned with the cartridge **12** before engagement. When the lancet **100** is not properly aligned with the cartridge **12,** the extensions **304** contact the outer surface **148a** of the bottom wall **148** which prevents the lancet **100** from deploying the needles **110.** When the lancet **100** is properly aligned with the cartridge **12,** the extensions **304** extend into the openings **138 (****Fig. 27****).** In this position the electrical contacts **132** and the electrical contacts **320** become physically engaged, e.g., they touch one another, and the lancet **100** is able to deploy the needles **110** to draw a physiological sample from a subject.

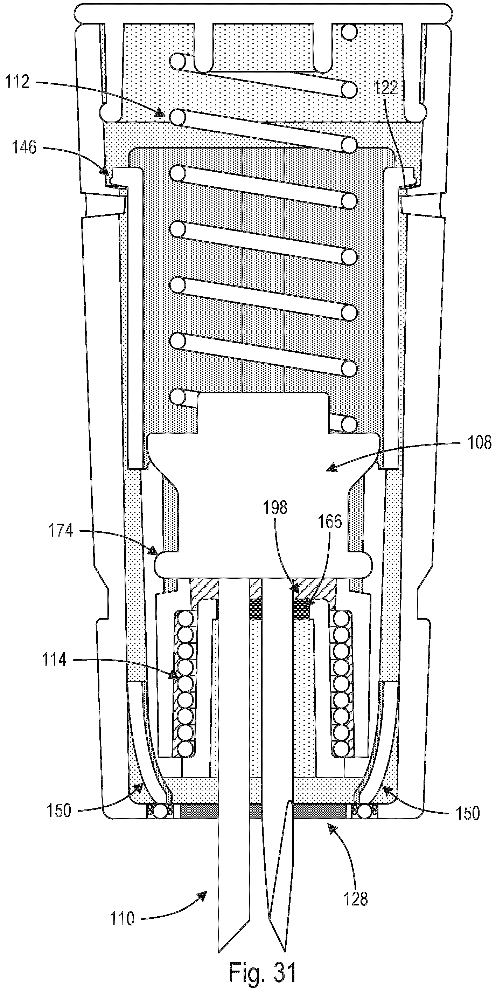

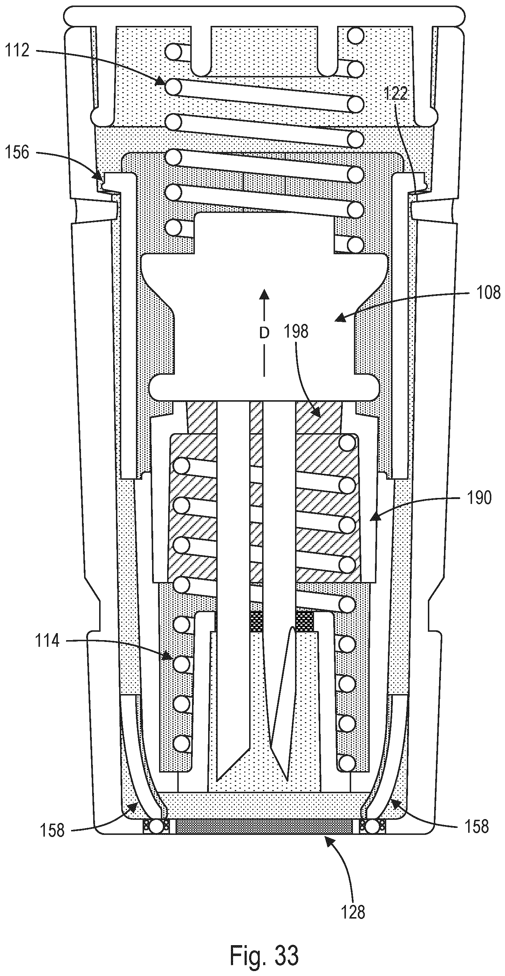

The engagement of the lancet **100** with the cartridge **12** causes the needles **110** of the lancet **100** to automatically move from an undeployed position **(****Fig. 3****)** to a deployed position **(****Fig. 31****)** and automatically from the deployed position to a retracted position **(****Fig. 33****).** Furthermore, when the lancet **100** engages with the base **300,** the hooks **318** of the locking members **316** couple to the groove **130** of the housing **102** via a snap fitting. This coupling causes the generation of an audible clicking sound that indicates to a user that the lancet **100** was correctly inserted into the cartridge **12.**

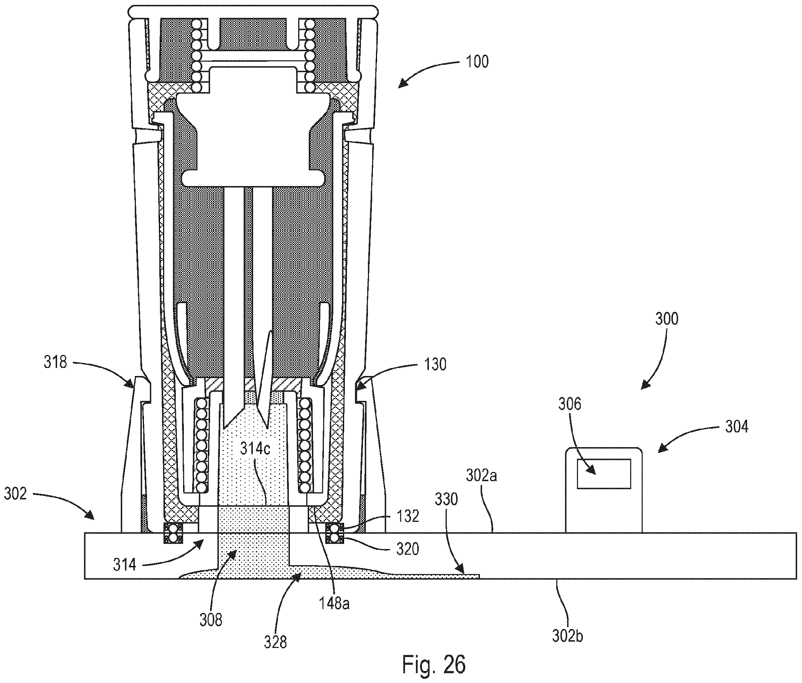

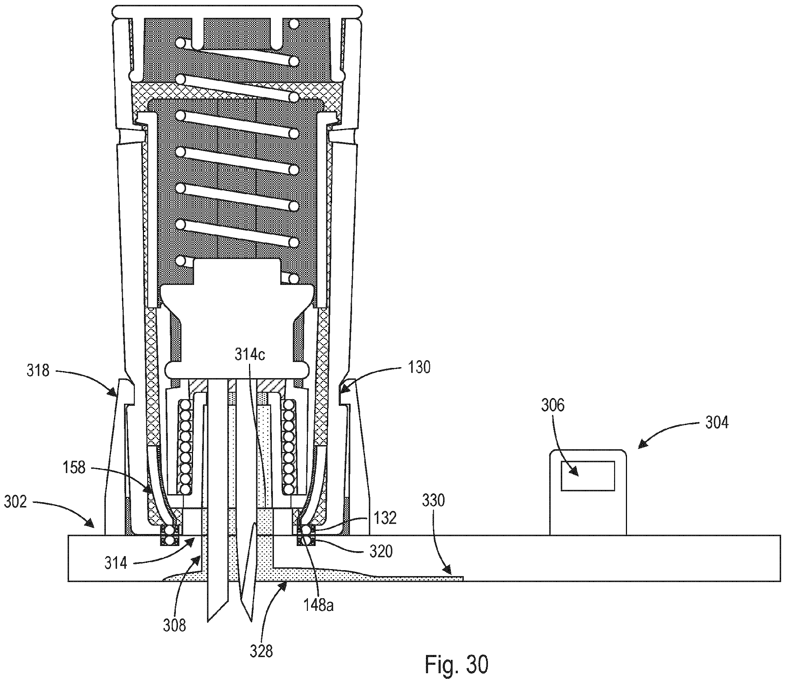

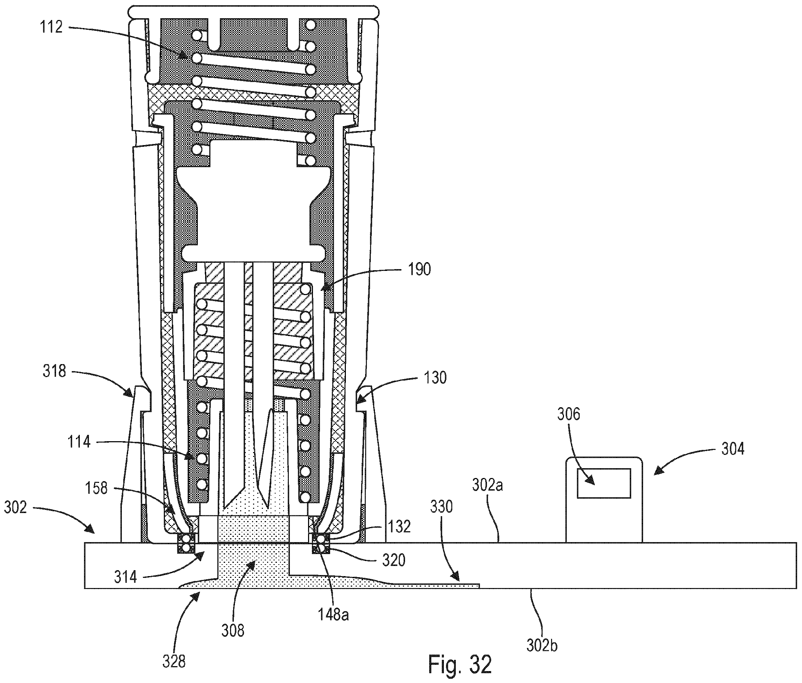

Upon engagement with the cartridge **12,** the electrical contacts **132** and the springs **136** are compressed into the contact receptacles **134** by the electrical contacts **320.** When the electrical contacts **320** are also pogo style connectors, the electrical contacts **320** and the springs **326** are also compressed into the contact receptacles **324.** While **Figs. 26****,** **30****,** and **32** depict the electrical contacts **320** as pogo style connectors, it is understood that the electrical contacts **320** may be electrically conductive pads as depicted in **Figs. 19** **and** **20****.** Furthermore, while **Figs. 21** and **22** depict the bottom wall **118** as including the electrical contacts **132** in other embodiments, as depicted in **Figs. 21** and **22****,** the side wall **116** can include the electrical contacts **132.** In some such embodiments, the hooks **318** include the electrical contacts **320.**

When the lancet **100** is coupled to the base **300** the inner circular projection **314** of the lancet receiving element **310** extends through the aperture **128** of the housing **102** and contacts the bottom wall **148** of the inner sleeve **106.** Specifically, the top surface **314c** of the inner circular projection **314** contacts the outer surface **148a** of bottom wall **148** which forces the inner sleeve **106** to move vertically upward in the direction of arrow **A** within the housing **102.** As previously discussed herein, grooves **124** of the housing **102** and the protrusions **162** of the inner sleeve **106** guide the vertical movement of the inner sleeve **106.** The vertical movement of the inner sleeve **106** causes the hooks **156** to disengage from the openings **126** and causes the extensions **152** to extend vertically above and rest upon the ledge **122.** In this position, the extensions **152** decompress and expand outwardly in the direction of arrow **B.**

When the extensions **152** expand, the needle platform **108** no longer rests upon the extensions **152** and hence can move vertically downward in the direction of arrow **C.** As the needle platform **108** moves downward, the injection spring **112** is allowed to expand and the force applied by the injection spring **112** causes the needle platform **108** (and therefore the needles **110)** to travel at a rate that is sufficient to cause the needles **110** to puncture the skin of a subject wearing the dermal patch system **10.** The expansion of the injection spring **112** causes the needles **110** to move and extend through the spring sleeve **190** via the opening **198,** through the post **168** via the opening **174,** through the opening **166** of the inner sleeve **106** and exit the housing **102** via the aperture **128** and pass through the base **300** of the cartridge **12** via the needle aperture **308.** Stated another way, the injection spring **112** moves the needles **110** to the deployed position.

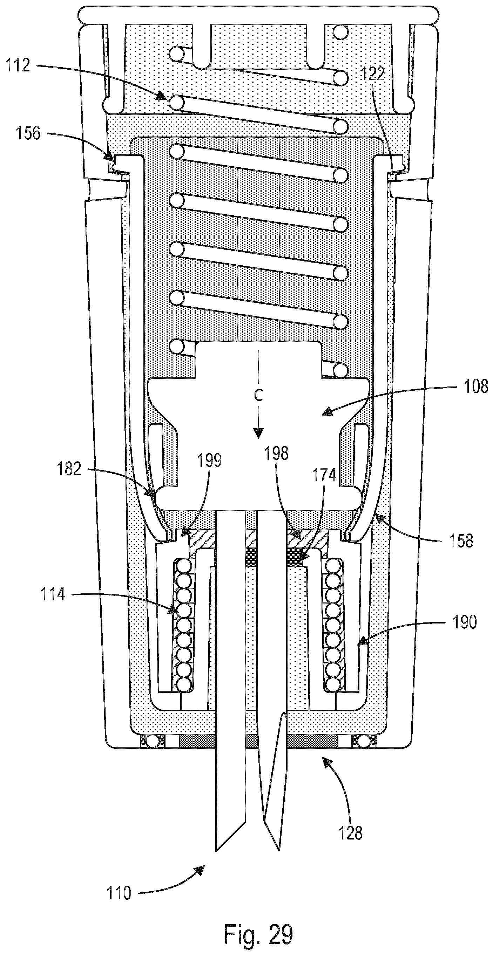

As previously discussed herein, the grooves **164** of the inner sleeve **106** and the projections **188** of the needle platform **108** guide the vertical movement of the needle platform **108.** As the needle platform moves in the direction of arrow **B,** the lower circular extension **182** contacts the deformable tabs **158 (****Fig. 29****).** This contact causes the tabs **158** to deflect away from the spring sleeve **190** of the lancet **100** or causes the tabs **158** to break. As depicted in **Fig. 31****,** when the tabs **158** break, the broken piece(s) of the tabs **158** fall into a void (receptacle) between the inner sleeve **106** and the housing **102.** In the deployed position, the needles **110** extend beyond the housing **102** and the lower circular extension **182** contacts the top wall **194** of the spring sleeve **190.**

When the tabs **158** deflect away from the spring sleeve **190** or break, the tabs **158** no longer contact the ledge **199** of the spring sleeve **190.** As a result, the retraction spring **114** is allowed to expand. The expansion of the retraction spring **114** moves the spring sleeve **190** vertically upward in the direction of arrow **D.** If the tabs **158** deflect and do not break, the spring sleeve **190** prevents the tabs **158** from contacting the retraction spring **114** as the retraction spring **114** expands. The spring sleeve **190** moves the needle platform **108** in the direction of arrow **D** to place the lancet **100** in the retracted position. That is, after moving to the deployed position, the retraction spring **114** causes the needles **110** to retract back into the inner volume **150** of the inner sleeve **106** via the needle aperture **308** of the base **300,** the aperture **128** of the housing **102,** and the opening **166** of the inner sleeve **106.** That is, after penetrating the skin of a subject, the retraction spring **114** causes the needles **110** to automatically retract back into the housing of the lancet **100** thereby placing the lancet **100** in the retracted position.

In this embodiment, the three needles **110** have substantially the same length, and by simultaneously moving the three needles **110** to the deployed position, the lancet **100** punctures the skin of the subject at three locations at the same time, though in other embodiments, two or all of the three needles **110** may have different lengths.

In some embodiments, the use of three needles **110** results in placing a portion of the skin under tension, which can in turn facilitate drawing blood through on or more puncture sites caused by the needles. In particular, as noted above, it has been discovered that the use of three needles **110** can advantageously allow a larger volume of blood to be drawn compared to the use of one, two, or a greater number of needles **110.** Without being limited to any particular theory, in some embodiments, in addition to placing a skin portion under tension, the use of three needles **110** may also improve the likelihood that at least one of the needles would cut through a capillary to extract a blood volume from the subject. Each of the needles **110** has a tapered tip that points outward (away from the center of the lancet) to maximize a distance between puncture locations on the subject's skin. Furthermore, the three needles **110** may be positioned such that the needle tips form an equilateral triangle and are therefore equally space from one another.

It is understood that the lancet **100** may be used with any cartridge of a dermal patch system that is configured to engage with the lancet **100.** That is, the lancet **100** may be used with any cartridge that includes an extension that causes the lancet **100** to transition from the undeployed position to the deployed position.

After the needles **110** retract, a physiological sample pools within the physiological sample well **328** of the base **300.** Due to gravity, wicking, and capillary action, the physiological sample travels from the physical sample well **328** to the sample collection reservoir **332** via the physiological sample channel **330.** A user can view the sample collection reservoir **332** via the viewing aperture **206** to ensure the physiological sample has traveled to the sample collection reservoir **332.** After drawing the physiological sample, the cartridge **12** may be removed from the skin of the subject and sent to a laboratory for analysis. A medical professional may then extract the physiological sample and analyze the sample.

As previously discussed herein, when the lancet **100** is properly engaged with the cartridge **12,** the electrical contacts **132** engage the electrical contacts **320.** When the electrical contacts **132** and the electrical contacts **320** are engaged, a closed electrical circuit is established between the biometric sensor **210,** the computer system **334** and the power supply **336.** In one embodiment, the cover **200** can include a power button (not shown) that when the lancet **100** is engaged with the cartridge **12** and the power button is pushed, the power supply **336** powers the biometric sensor **210** and the computer system **334.** In another embodiment, the power supply **336** is a charged capacitor, when the circuit is closed, the power supply **336** automatically powers the biometric sensor **210** and the computer system **334.**

In embodiments in which the biometric sensor **210** is a fingerprint scanner, while a user obtains the physiological sample from a subject, the user or subject can place their finger on the fingerprint scanner **210** such that the user's or subject's fingerprint data can be collected. When powered, the biometric sensor **210** obtains the user's or subject's fingerprint data and sends the fingerprint data to the computer system **334,** where it can be stored in a memory module of the computer system **334.** The biometric sensor **210** also sends the date and the time that the biometric data, in this embodiment fingerprint data, to the computer system **334.** The stored fingerprint data, the date, and the time can be used to establish a chain of custody of the cartridge **12** and therefore the drawn physiological sample. That is, the stored fingerprint data can be matched to a fingerprint of the user or the subject at a later time to establish that the subject was subject was the individual from whom the physiological sample was drawn or the user was the individual that drew the physiological sample from the subject. For example, the memory of the computer system may be accessed to obtain the stored fingerprint data and comparted to other fingerprint data that was obtained before or after the fingerprint data stored in the memory of the computer system. This data may be compared using various processing systems.

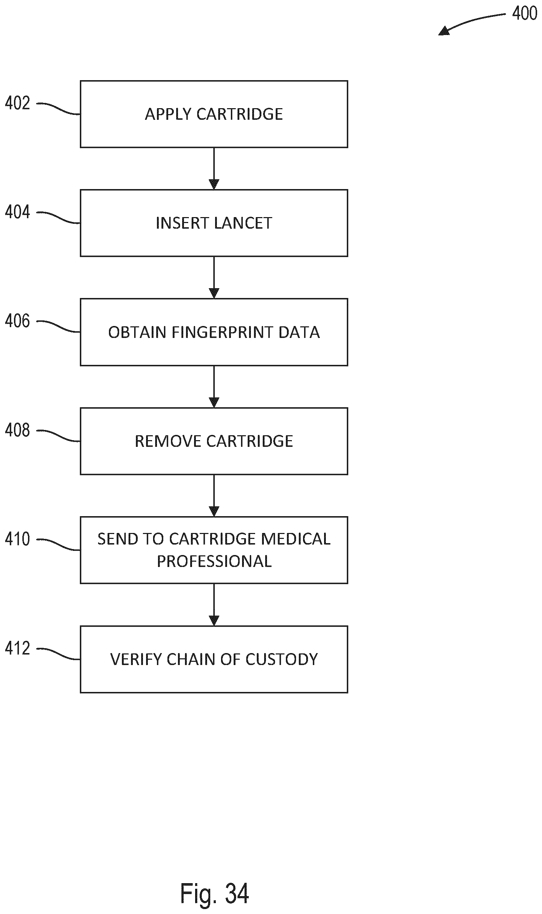

Referring now to **Fig. 34****,** a method **400** for obtaining a physiological sample from a subject is disclosed herein.

At **402,** a user attaches the cartridge **12** of the dermal patch system **10** to the skin of a subject at a suitable location (e.g., arm, leg, etc.) as previously discussed herein.

At **404,** the lancet **100** engages with the cartridge **12** to draw the physiological sample and allow the power supply **336** to power the biometric sensor **210** and the computer system **334** as previously discussed herein.

At **406,** the biometric sensor **210** obtains fingerprint data from the user of the dermal patch system **10** and sends a signal indicative of the fingerprint data to the computer system **334** as previously discussed herein. In response to receiving the signal indicative of the fingerprint data, the computer system **334** stores the fingerprint data in a memory of the computer system **334.**

At **408,** the cartridge **12** is removed from the skin of the subject as previously discussed herein.

At **410,** the cartridge **12** is sent to a medical professional to analyze the drawn physiological sample as previously discussed herein.

At **412,** a chain of custody of the cartridge **12** is established by verifying an individual's fingerprint matches the fingerprint data stored in the memory of the computer system **334.**

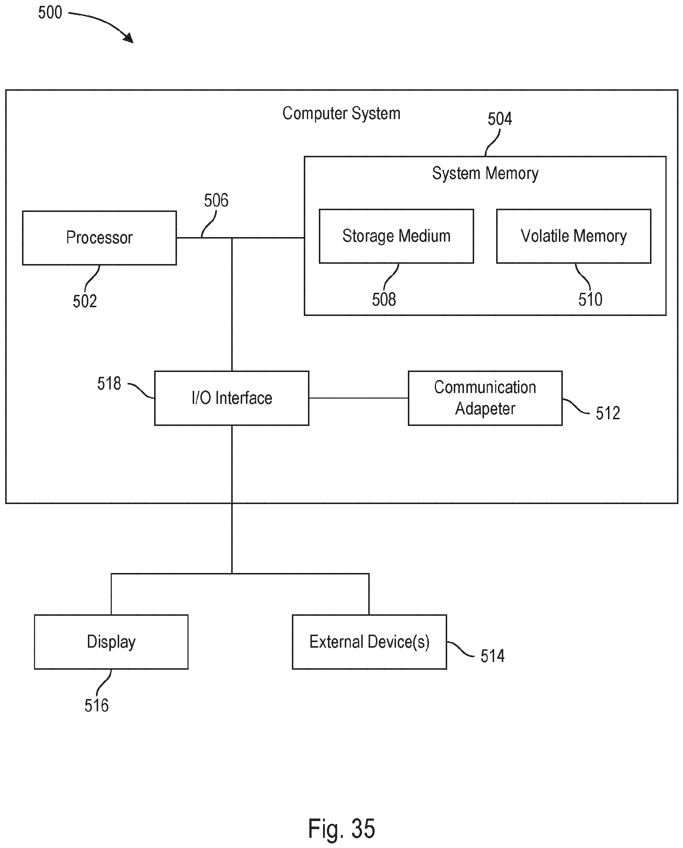

Referring now to **Fig. 35****,** a computer system **500** is shown in accordance with an exemplary embodiment. The computer system **500** may serve as any computer system disclosed herein. As used herein a computer system (or device) is any system/device capable of receiving, processing, and/or sending data. Computer systems include, but are not limited to, microprocessor-based systems, a system on a chip ("SOC") personal computers, servers, hand-held computing devices, tablets, smartphones, multiprocessor-based systems, mainframe computer systems, virtual reality ("VR") headsets and the like.

As shown in **Fig. 35****,** the computer system **500** includes one or more processors or processing units **502,** a system memory **504,** and a bus **506** that couples the various components of the computer system **500** including the system memory **504** to the processor **502.** The system memory **504** includes a computer readable storage medium **508** and volatile memory **510** (e.g., Random Access Memory, cache, etc.). As used herein, a computer readable storage medium includes any media that is capable of storing computer readable; program instructions and is accessible by a processor. The computer readable storage medium **508** includes non-volatile and non-transitory storage media (e.g., flash memory, read only memory (ROM), hard disk drives, etc.). Computer program instructions as described herein include program modules (e.g., routines, programs, objects, components, logic, data structures, etc.) that are executable by a processor. Furthermore, computer readable program instructions, when executed by a processor, can direct a computer system to function in a particular manner such that a computer readable storage medium comprises an article of manufacture. Specifically, the computer readable program instructions when executed by a processor can create a means for carrying out at least a portion of the steps of the methods disclosed herein.

The bus **506** may be one or more of any type of bus structure capable of transmitting data between components of the computer system **500** (e.g., a memory bus, a memory controller, a peripheral bus, an accelerated graphics port, etc.).

The computer system **500** may further include a communication adapter **512** which allows the computer system **500** to communicate with one or more other computer systems/devices via one or more communication protocols (e.g., Wi-Fi, BTLE, etc.) and in some embodiments may allow the computer system **500** to communicate with one or more other computer systems/devices over one or more networks (e.g., a local area network (LAN), a wide area network (WAN), a public network (the Internet), etc.).

In some embodiments, the computer system **500** may be connected to one or more external devices **514** and a display **516.** As used herein, an external device includes any device that allows a user to interact with a computer system (e.g., mouse, keyboard, touch screen, etc.). An external device **514** and the display **516** may be in communication with the processor **502** and the system memory **504** via an Input/Output (I/O) interface **518.**

The display **516** may display a graphical user interface (GUI) that may include a plurality of selectable icons and/or editable fields. A user may use an external device **514** (e.g., a mouse) to select one or more icons and/or edit one or more editable fields. Selecting an icon and/or editing a field may cause the processor **502** to execute computer readable program instructions stored in the computer readable storage medium **508.** In one example, a user may use an external device **514** to interact with the computer system **500** and cause the processor **502** to execute computer readable program instructions relating to at least a portion of the steps of the methods disclosed herein.

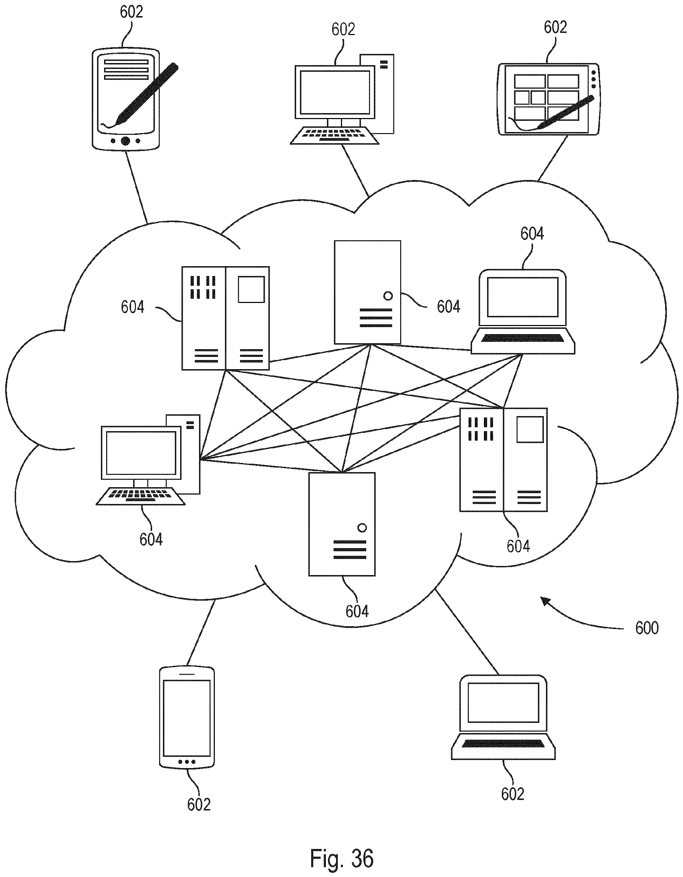

Referring now to **Fig. 36****,** a cloud computing environment **600** is depicted in accordance with an exemplary embodiment. The cloud computing environment **600** is connected to one or more user computer systems **602** and provides access to shared computer resources (e.g., storage, memory, applications, virtual machines, etc.) to the user computer systems **602.** As depicted in **Fig. 36****,** the cloud computing environment includes one or more interconnected nodes **604.** Each node **604** may be a computer system or device local processing and storage capabilities. The nodes **604** may be grouped and in communication with one another via one or more networks. This allows the cloud computing environment **600** to offer software services to the one or more computer services to the one or more user computer systems **602** and as such, a user computer system **602** does not need to maintain resources locally.

In one embodiment, a node **604** includes computer readable program instructions for carrying out various steps of various methods disclosed herein. In these embodiments, a user of a user computer system **602** that is connected to the cloud computing environment may cause a node **604** to execute the computer readable program instructions to carry out various steps of various methods disclosed herein.

As previously discussed, the above may be implemented by way of computer readable instructions, encoded or embedded on computer readable storage medium (which excludes transitory medium), which, when executed by a processor(s), cause the processor(s) to carry out the methods of the present disclosure.

While various embodiments have been illustrated and described in detail in the drawings and foregoing description, such illustration and description are to be considered illustrative or exemplary and not restrictive; embodiments of the present disclosure are not limited to the disclosed embodiments. Other variations to the disclosed embodiments can be understood and effected by those skilled in the art in practicing embodiments of the present disclosure, from a study of the drawings, the disclosure, and the appended claims.

In the claims, the word "comprising" does not exclude other elements or steps, and the indefinite article "a" or "an" does not exclude a plurality. A single processor or other processing unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

The disclosure also comprises the following aspects:
1. A dermal patch system for collecting a physiological sample from a subject, comprising:
   a cartridge configured to attach to the skin of the subject, and
   a lancet configured to engage with the cartridge and draw a physiological sample upon engagement,
      wherein the cartridge includes a biometric sensor configured to obtain biometric data from a subject.
2. The dermal patch system of aspect 1, wherein the cartridge further includes:
   a computer system configured to receive the biometric data from the and store the biometric data within a memory of the computer system.
3. The dermal patch system of any of the preceding aspects, wherein the cartridge further includes:
   a power supply configured to power the biometric sensor and the computer system.
4. The dermal patch system of any of the preceding aspects, wherein the power supply is configured to power the biometric sensor and the computer system when the lancet engages with the cartridge.
5. The dermal patch system of any of the preceding aspects, wherein the power supply is connected to the biometric sensor and the computer system such that a closed circuit is established only in response to the engagement of the lancet with the cartridge.
6. The dermal patch system of any of the preceding aspects, wherein the cartridge includes first electrical contacts and the lancet includes second electrical contacts, wherein the engagement of the lancet with the cartridge results in engagement of the first and second electrical contacts so as to establish said closed circuit.
7. The dermal patch system of any of the preceding aspects, wherein the first and second electrical contacts are pogo style connectors.
8. The dermal patch system of any of the preceding aspects, wherein the first electrical contacts are electrically conductive pads and the second electrical contacts are pogo style connectors.
9. The dermal patch system of any of the preceding aspects, wherein the lancet includes a needle and wherein the cartridge includes a lancet receiving element configured to automatically cause the lancet to deploy the needle to draw a physiological sample upon engagement with the lancet.
10. The dermal patch system of aspect 9, wherein the lancet is configured to automatically retract the needles.
11. The dermal patch system of aspect 9 or 10, wherein the lancet receiving element includes the first electrical contacts and a portion of the lancet that engages with the lancet receiving element includes the second electrical contacts.
12. The dermal patch system of any of the preceding aspects, wherein the power supply comprises a battery or a charged capacitor.
13. The dermal patch system of any of the preceding aspects, wherein the power supply is a charged capacitor that automatically powers the biometric sensor and the computer system when the lancet engages with the cartridge.
14. The dermal patch system of any of the preceding aspects, wherein the cartridge is configured to receive and store the physiological sample.
15. The dermal patch system of any of the preceding aspects, wherein the biometric sensor is a fingerprint scanner or an iris scanner.
16. A method for obtaining a physiological sample from a subject, comprising:
   affixing a cartridge of a dermal patch system to the skin of a subject,
   engaging a lancet with the cartridge to draw a physiological sample, and
   obtaining biometric data of the subject during at least a portion of temporal period associated with drawing the physiological.
17. The method of aspect 16, further comprising:
   storing the drawn physiological sample within the cartridge.
18. The method of aspect 16 or 17, further comprising:
   storing the fingerprint data in a memory of a computer system that is disposed within the cartridge.
19. The method of any of aspects 16 to 18, wherein the cartridge includes a power supply that is configured to power the biometric sensor and the computer system upon engagement of the lancet with the cartridge.
20. The method of aspect 19, wherein the power supply is a charged capacitor and engaging the lancet with the cartridge closes a circuit which causes the charged capacitor to automatically power the biometric sensor and the computer system.

## Claims

1. A dermal patch system for collecting a physiological sample from a subject, comprising:
a cartridge configured to attach to the skin of the subject, and
a lancet configured to engage with the cartridge and draw a physiological sample upon engagement,
wherein the cartridge includes a biometric sensor configured to obtain biometric data from a subject.

2. The dermal patch system of claim 1, wherein the cartridge further includes:
a computer system configured to receive the biometric data from the and store the biometric data within a memory of the computer system.

3. The dermal patch system of claim 2, wherein the cartridge further includes:
a power supply configured to power the biometric sensor and the computer system.

4. The dermal patch system of claim 3, wherein the power supply is configured to power the biometric sensor and the computer system when the lancet engages with the cartridge.

5. The dermal patch system of claim 4, wherein the power supply is connected to the biometric sensor and the computer system such that a closed circuit is established only in response to the engagement of the lancet with the cartridge.

6. The dermal patch system of claim 5, wherein the cartridge includes first electrical contacts and the lancet includes second electrical contacts, wherein the engagement of the lancet with the cartridge results in engagement of the first and second electrical contacts so as to establish said closed circuit.

7. The dermal patch system of claim 6, wherein the lancet includes a needle and wherein the cartridge includes a lancet receiving element configured to automatically cause the lancet to deploy the needle to draw a physiological sample upon engagement with the lancet.

8. The dermal patch system of claim 7, wherein the lancet is configured to automatically retract the needles.

9. The dermal patch system of claim 7, wherein the lancet receiving element includes the first electrical contacts and a portion of the lancet that engages with the lancet receiving element includes the second electrical contacts.

10. The dermal patch system of claim 1, wherein the power supply is a charged capacitor that automatically powers the biometric sensor and the computer system when the lancet engages with the cartridge.

11. The dermal patch system of claim 1, wherein the cartridge is configured to receive and store the physiological sample.

12. The dermal patch system of claim 1, wherein the biometric sensor is a fingerprint scanner or an iris scanner.

13. A method for obtaining a physiological sample from a subject, comprising:
affixing a cartridge of a dermal patch system to the skin of a subject,
engaging a lancet with the cartridge to draw a physiological sample, and
obtaining biometric data of the subject during at least a portion of temporal period associated with drawing the physiological.

14. The method of claim 13, further comprising:
storing the drawn physiological sample within the cartridge.

15. The method of claim 13, further comprising:
storing the fingerprint data in a memory of a computer system that is disposed within the cartridge.
